(19) Europäisches Patentamt
European Patent Office
Office européen des brevets

(11) EP 3 485 920 B1

(12) **EUROPÄISCHE PATENTSCHRIFT**

(45) Veröffentlichungstag und Bekanntmachung des Hinweises auf die Patenterteilung:
**20.05.2020 Patentblatt 2020/21**

(51) Int Cl.:
***A61L 29/16*** *(2006.01)* ***A61L 29/18*** *(2006.01)*

(21) Anmeldenummer: **18213686.1**

(22) Anmeldetag: **09.02.2007**

(54) **FALTENBALLONBESCHICHTUNGSVERFAHREN**

COATING METHOD FOR A FOLDED BALLOON

PROCÉDÉ DE REVÊTEMENT DE BALLONNET REPLIABLE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **09.02.2006 DE 102006006067**

(43) Veröffentlichungstag der Anmeldung:
**22.05.2019 Patentblatt 2019/21**

(62) Dokumentnummer(n) der früheren Anmeldung(en) nach Art. 76 EPÜ:
**16197537.0 / 3 150 236**
**07721910.3 / 1 981 559**

(73) Patentinhaber: **B. Braun Melsungen AG**
**34212 Melsungen (DE)**

(72) Erfinder:
- **HEITZMANN, Christoph**
  **12459 Berlin (DE)**
- **VON HOLST, Armin**
  **12247 Berlin (DE)**

(74) Vertreter: **Patentanwälte**
**Ruff, Wilhelm, Beier, Dauster & Partner mbB**
**Kronenstraße 30**
**70174 Stuttgart (DE)**

(56) Entgegenhaltungen:
**WO-A-2004/028582** **WO-A2-2004/028610**
**WO-A2-2008/086794**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 485 920 B1

## Beschreibung

Die vorliegende Erfindung betrifft ein Verfahren zur Beschichtung oder Befüllung der Falten eines Katheterfaltenballons.

Zur Behandlung von Stenosen ist es Stand der Technik, Stents zu setzen oder durch sogenanntes biologisches Stenting die verengte Gefäßregion wieder aufzuweiten. Dazu werden oftmals Stents und/oder Katheterballons verwendet, welche mit einem Polymer als Wirkstoffträger und einem Anti-Restenose-Wirkstoff beschichtet sind.

Da in der Regel der Katheterballon mit oder ohne aufgesetztem, d.h. gekrimptem Stent eine längere Gefäßstrecke passieren muss, um zum Ort der Gefäßverengung zu gelangen, stellt sich das Problem, den Wirkstoff vor führzeitiger Ablösung von dem Stent und/oder Ballon zu schützen.

Bezüglich der Stents hat man bereits diverse Lösungsvorschläge für dieses Problem vorgeschlagen. So offenbaren US 2004/0071861 und WO 03/035131 A beispielsweise, dass in das Gittergerüst des Stent kleine Kavitäten zur Aufnahme eines Wirkstoffs vorgesehen werden können und man den Wirkstoff in den Kavitäten durch das Aufbringen einer Schutzbeschichtung schützen kann.

WO 02/45744 A beschreibt einen Stent ohne Kavitäten, der mit einer ersten Beschichtung umfassend einen Wirkstoff und einer darüber liegenden unelastischen Deckschicht versehen ist, welche den Wirkstoff schützt. Die Erfindung gemäß WO 02/45744 A bestehe nun darin, dass bei der Expansion des Stent die unelastische Deckschicht aufbricht und den Wirkstoff gezielt am Ort seiner Bestimmung freisetzt.

Für Katheterballons sind derartige Deckschichten auch denkbar, aufgrund der großen Fläche des Katheterballons jedoch als praktikable Ausführungsformen schwerer zu realisieren.

Das europäische Patent EP 0 519 063 B1 offenbart die Möglichkeit, einen Faltenballon mit Mikrokapseln zu beschichten, wobei in den Mikrokapseln ein pharmakologischer Wirkstoff eingeschlossen sein kann. Ferner offenbart EP 0 519 063 B1 die Möglichkeit, einen Teil der Mikrokapseln in den Falten des Katheterballon einzuschließen, wenn der Katheterballon im expandierten, d.h. im inflatierten Zustand mit den Mikrokapseln bestäubt und danach wieder zusammengefaltet, d.h. deflatiert wird (s. Anspruch 8 von EP 0 519 063 B1).

Diese Ausführungsform weist jedoch weiterhin das Problem auf, dass nur ein Teil der Mikrokapseln in den Falten eingeschlossen wird und somit die in den Falten befindliche Wirkstoffmenge nicht bekannt ist. Die nicht in den Falten eingeschlossenen Mikrokapseln werden beim Einführen des Katheterballons fast vollständig von der Ballonoberfläche gelöst und gelangen nicht zum Bestimmungsort. Zudem ist das Beschichtungsverfahren gemäß EP 0 519 063 B1 nur auf Feststoffe und insbesondere Mikrokapseln beschränkt und kann nicht auf Flüssigkeiten angewendet werden.

Verfahren zum Beschichten von Ballonkathetern durch Eintauchen in eine Wirkstofflösung sind aus der WO 2004/028582 A1 sowie WO 2004/028610 A2 bekannt. Aus der WO 2008/086794 A2 ist ein Verfahren zur Beschichtung eines Katheterballons mit einer definierten Menge eines pharmakologischen Wirkstoffs bekannt, bei welchem eine Beschichtungsvorrichtung mit einer Volumenmesseinrichtung zur Abgabe einer messbaren Menge einer Beschichtungslösung mittels einer Abgabevorrichtung gezielt auf die Oberfläche des Katheterballons verwendet wird.

Die Aufgabe der vorliegenden Erfindung bestand darin, bei der Wirkstoffapplikation via Katheterballon den Wirkstoff derart aufzutragen, dass eine ungewünschte vorzeitige Ablösung nicht eintritt.

Diese Aufgabe wird durch ein Beschichtungsverfahren gemäß Patentanspruch 1 gelöst. Weitere vorteilhafte Ausgestaltungen, Aspekte und Details der Erfindung ergeben sich aus den abhängigen Ansprüchen, der Beschreibung, den Beispielen und den Figuren.

Die Beschichtung oder Befüllung der Falten erfolgt mit einem flüssigen Gemisch umfassend zumindest einen Wirkstoff und ein Lösungsmittel oder einen Trägerstoff. Des weiteren können noch Kontrastmittel, Salze, Hilfsstoffe oder andere pharmakologisch verträgliche Stoffe anwesend sein.

Als Wirkstoffe können beliebige antiproliferative, antiinflammatorische, antiphlogistische, zytostatische, zytotoxische, antiangiogene, antirestenotische oder antithrombotische Wirkstoffe eingesetzt werden.

Beispiele derartiger Wirkstoffe sind Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat,

Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2ω, Mitoxanthrone, Mycophenolatmofetil, c-myc-Antisense, b-myc-Antisense, β-Lapachon,Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, die Muskelzellproliferation hemmende monoklonale Antikörper, bFGF-Antagonisten, Probucol, Prostaglandine, 1-Hydroxy-11-Methoxycanthin-6-on, Scopolectin, NO-Donatoren, Pentaerythrityltetranitrat, Syndnoeimine, S-Nitrosoderivate, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel, Derivate und Analog des Paclitaxel, 6-α-Hydroxy-Paclitaxel, 2'-Succinylpaclitaxel, 2'-Succinylpaclitaxeltriethanolamin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere, Kohlensuboxids (MCS), macrocyclische Oligomere von Kohensuboxid, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, in Virenüberträger inkorporierte Nukleinsäuren, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Enoxoparin, desulfatiertes und N-reacetyliertes Heparin, Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon a, β und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin sowie Gemische der vorgenannten Wirkstoffe.

Insbesondere bevorzugte Wirkstoffe sind Rapamycin (Sirolimus) und Paclitaxel, Derivate und Analog des Paclitaxel, 6-α-Hydroxy-Paclitaxel, 2'-Succinylpaclitaxel, 2'-Succinylpaclitaxeltriethanol-amin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere sowie Gemische der vorgenannten Wirkstoffe. Insbesondere bevorzugt ist Paclitaxel.

Natürlich können auch Wirkstoffgemische eingesetzt werden wie beispielsweise ein Wirkstoffgemisch aus Paclitaxel und Trapidil oder aus Paclitaxel mit einem NO-Donor oder aus Rapamycin mit Vitamin A oder Vitamin C.

Auch wichtig ist das verwendete Lösungsmittel, welches nach Befüllung der Falten durch Trocknung bei Normaldruck oder im Vakuum entfernt wird.

Als Lösungsmittel können leichtflüchtige organische Verbindungen verwendet werden wie beispielsweise Dichlormethan, Chloroform, Ethanol, Aceton, Heptan, n-Hexan, DMF, DMSO, Methanol, Propanol, Tetrahydrofuran (THF), Methylenchlorid, Ether, Petrolether, Essigsäureethylester, Cyclohexan. Bei der Wahl des Lösungsmittel ist es vor allem wichtig, dass es das Material des Katheterballons nicht angreift bzw. unbrauchbar macht bzw. die Kontaktzeit des Lösungsmittels so gering ist, dass keine Schädigung entstehen kann bzw. eine eventuelle Schädigung unerheblich ist und nicht zu Schädigungen führt, welche bei der Dilatation ein Platzen des Ballons verursachen können.

Als Lösungsmittel haben sich vorzugsweise Alkohole und insbesondere Diole und Triole auch in Kombination mit Monoolen bewährt. Das bevorzugte Lösungsmittel kann vorzugsweise aus folgender Gruppe ausgewählt werden: Methanol, Ethanol, Iospropanol, n-Butanol, iso-Butanol, t-Butanol, Ethylenglykol, Propylenglykol, 1,3-Propandiol, Butylenglykol, 1,3-Butandiol, 1,4-Butandiol, Glycerin, 1,2,3-Butantriol, 1,2,4-Butantriol und 1,2,3,4-Butantetraol, wobei auch Gemische dieser Lösungsmittel sowie Gemische mit den vorher genannten organischen Lösungsmitteln eingesetzt werden können. Bevorzugt sind Ethylenglykol, Propylenglykol, 1,3-Propandiol, Butylenglykol, 1,3-Butandiol, 1,4-Butandiol und insbesondere bevorzugt ist Glycerin.

Auch Wasser kann im Lösungsmittelgemisch enthalten sein, jedoch vorzugsweise in einer Menge geringer als 50 Gew.-%, vorzugsweise 30 Gew.-% und insbesondere bevorzugt 10 Gew.-% bezogen auf die Gesamtlösung.

Je nach erfindungsgemäßem Beschichtungsverfahren werden dünnviskose Lösungen des Wirkstoffs oder dickviskose Lösungen des Wirkstoffs oder der Wirkstoffkombination benötigt.

Viskositätsmessungen sind für einen Fachmann gängige Praxis und erfolgen vorzugsweise mit Viskosimetern. Die Viskosität muss je nach Art und Ausgestaltung der Falten eingestellt werden.

Diverse Faltentypen und Typen und Ausführungsformen von Faltenballons sind beispielsweise in EP 0 519 063 B1, WO 94/23787 A1 oder WO 03/059430 A1 offenbart. Da jedoch fast jeder deflatierbare bzw. dilatierbare Katheterballon Falten aufweist, sind die erfindungsgemäßen Beschichtungsverfahren grundsätzlich auf jeden aufblasbaren Katheterballon und nicht nur auf die in WO 94/23787 A1 oder WO 03/059430 A1 genannten speziellen Ausführungsformen beschränkt.

Als Lösungsmittel können neben den oben genannten oder zusammen mit den oben genannten Lösungsmitteln auch Öle, Fettsäuren und Fettsäureester verwendet werden. Bevorzugte Öle sind beispielsweise: Leinöl, Flachsöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran und/oder Mischungen der vorgenannten Öle eingesetzt.

Unverdünnte Öle werden dann als Lösungsmittel eingesetzt, wenn eine dickviskose Beschichtungslösung benötigt wird. Durch Zugabe lipophiler Lösungsmittel kann die Viskosität erniedrigt und somit ein gewünschter Viskositätsgrad eingestellt werden. Andererseits können dünnviskose Lösungen durch Zugabe von Ölen oder Fetten in ihrer Viskosität erhöht werden.

Der Begriff "Lösung" oder "Beschichtungslösung" wie hierin verwendet soll nicht nur klare Lösungen bezeichnen sondern auch Emulsionen, Dispersionen und Suspensionen von einem oder mehreren Wirkstoffen in einem Lösungsmittel oder Lösungsmittelgemisch optional mit weiteren Trägern, Hilfsstoffen oder beispielsweise Kontrastmitteln.

Der Begriff "Zusammensetzung" oder "wirkstoffenthaltende Zusammensetzung" wie hierin verwendet soll nicht nur Lösungen bezeichnen sondern auch Emulsionen, Dispersionen, Suspensionen, Öle, Pasten und dickflüssige Mischungen enthaltend mindestens eine pharmakologischen Wirkstoff. Dennoch sind diese Zusammensetzungen nicht fest, sondern dünnflüssig bis dickflüssig oder gel- oder pastenförmig.

Als Öle oder grundsätzlich als lipophile Substanzen können natürliche und synthetische Öle, Fette, Lipide, Lipoide und Wachse eingesetzt werden.

WO 03/022265 A1 beschreibt beispielsweise ölige Formulierungen von Paclitaxel, welche ebenfalls eingesetzt werden können und bevorzugt sind. Vergleichbare Öllösungen lassen sich auch mit anderen Wirkstoffen wie beispielsweise Trapidil oder Rapamycin herstellen.

Weitere Beispiele für geeignete Öle oder lipophile Substanzen lassen sich durch folgende allgemeinen Formeln darstellen:

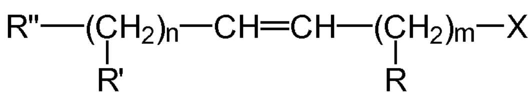

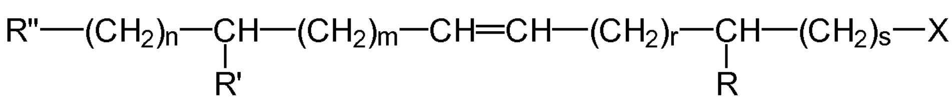

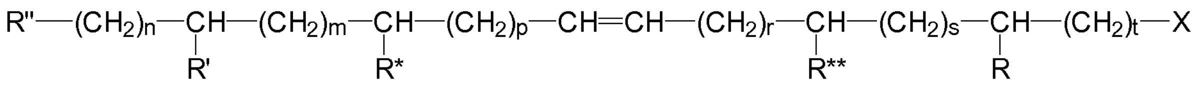

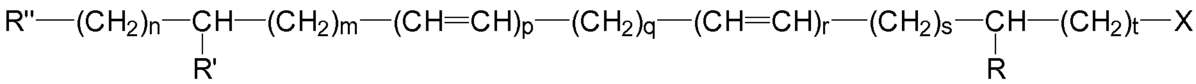

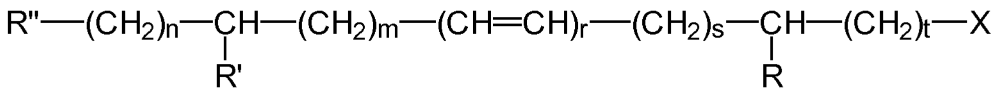

worin

R, R', R", R* und R** unabhängig voneinander für Alkyl-, Alenyl-, Alkinyl-, Heteroalkyl-, Cycloalkyl-, Heterocyclylreste mit 1 bis 20 Kohlenstoffatomen, Aryl-, Arylalkyl-, Alyklaryl-, Heteroarylreste mit 3 bis 20 Kohlenstoffatomen oder für funktionelle Gruppen stehen und bevorzugt folgende Reste bedeuten: -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-NO_2$, -F, -Cl, -Br, -I, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, $-COOCH_3$, $-COOC_2H_5$, $-COOC_3H_7$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, -OOC-cyclo-$C_3H_5$, $-OOC-CH(CH_3)_2$, $-OOC-C(CH_3)_3$, $-CONH_2$, $-CONHCH_3$, $-CONHC_2H_5$, $-CONHC_3H_7$, $-CON(CH_3)_2$, $-CON(C_2H_5)_2$, $-CON(C_3H_7)_2$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5$)$_2$, $-N[CH(CH_3)_2]_2$, $-N[C(CH_3)_3]_2$, $-SOCH_3$, $-SOC_2H_5$, $-SOC_3H_7$, $-SO_2CH_3$, $-SO_2C_2H_5$, $-SO_2C_3H_7$, $-SO_3H$, $-SO_3CH_3$, $-SO_3C_2H_5$, $-SO_3C_3H_7$, $-OCF_3$, $-OC_2F_5$, $-O-COOCH_3$, $-O-COOC_2H_5$, $-O-COOC_3H_7$, -O-COO-cyclo-$C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CO-N(CH_3)_2$, $-NH-CO-N(C_2H_5)_2$, $-O-CO-NH_2$, $-O-CO-NHCH_3$, $-O-CO-NHC_2H_5$, $-O-CO-NHC_3H_7$, $-O-CO-N(CH_3)_2$, $-O-CO-N(C_2H_5)_2$, $-O-CO-OCH_3$, $-O-CO-OC_2H_5$, $-O-CO-OC_3H_7$, -O-CO-O-cyclo-$C_3H_5$, $-O-CO-OCH(CH_3)_2$, $-O-CO-OC(CH_3)_3$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, $-CH_3$, $-C_2H_5$, $-C_3H_7$, -cyclo-$C_3H_5$, $-CH(CH_3)_2$, $-C(CH_3)_3$, $-C_4H_9$, $-CH_2-CH(CH_3)_2$, $-CH(CH_3)-C_2H_5$, -Ph, $-CH_2$-Ph, $-CPh_3$, $-CH=CH_2$, $-CH_2-CH=CH_2$, $-C(CH_3)=CH_2$, $-CH=CH-CH_3$, $-C_2H_4-CH=CH_2$, $-CH=C(CH_3)_2$, -C=CH, $-C\equiv C-CH_3$, $-CH_2-C\equiv CH$;

X für eine Estergruppe oder Amidgruppe und insbesondere für -O-alkyl, -O-CO-alykl, -O-CO-O-alkyl, -O-CO-NH-alkyl, -O-CO-N-dialkyl, -CO-NH-alkyl, -CO-N-dialkyl, -CO-O-alkyl, -CO-OH, -OH;

m, n, p, q, r, s und t unabhängig voneinander ganze Zahlen von 0 bis 20, bevorzugt von 0 bis 10 bedeuten.

Die Bezeichnung "alkyl" beispielsweise bei -CO-O-alkyl bedeutet vorzugsweise eine der für die vorgenannten Reste R, R' usw. genannten Alkylreste, z.B. $-CH_2$-Ph. Die Verbindungen der vorgenannten allgemeinen Formeln können auch in Form ihrer Salze, als Racemate oder Diastereomerengemische, als reine Enantiomeren oder Diastereomeren sowie als Gemische oder Oligomere oder Copolymere oder Blockcopolymere vorliegen. Ferner können die vorgenannten Verbindungen im Gemisch mit anderen Substanzen wie den biostabilen und biodegradierbaren Polymeren und insbesondere im Gemisch mit den hierin genannten Ölen und/oder Fettsäuren eingesetzt werden. Bevorzugt sind derartige Gemische und Einzelsubstanzen, welche zur Polymerisation, insbesondere zur Autopolymerisation geeignet sind.

Bevorzugt sind jedoch natürlich vorkommende Öle, Fettsäuren und Fettsäureester wie beispielsweise Ölsäure, Eicosapentaensäure, Timnodonsäure, Docosahexaensäure, Arachidonsäure, Linolsäure, $\alpha$-Linolensäure, $\gamma$-Linolensäure sowie Mischungen und Ester der vorgenannten Fettsäuren. Insbesondere bevorzugt sind Omega-9-Fettsäuren, Omega-3-Fettsäuren und Omega-6-Fettsäuren sowie deren Ester und Mischungen enthaltend diese Stoffe vorzugsweise mit einem Gewichtsanteil von mindestens 10 Gew.-%.

Weitere geeignete Fettsäuren sind in den Tabellen 1 bis 4 aufgeführt.

Tabelle 1: Monoolefinische Fettsäuren

| Systematischer Name | Trivialname | Kurzform |
|---|---|---|
| cis-9-Tetradecensäure | Myristoleinsäure | 14:1(n-5) |
| cis-9-Hexadecensäure | Palmitoleinsäure | 16:1(n-7) |
| cis-6-Octadecensäure | Petroselinsäure | 18:1(n-12) |
| cis-9-Octadecensäure | Ölsäure | 18:1(n-9) |

(fortgesetzt)

| Systematischer Name | Trivialname | Kurzform |
|---|---|---|
| cis-11-Octadecensäure | Vaccensäure | 18:1(n-7) |
| cis-9-Eicosensäure | Gadoleinsäure | 20:1(n-11) |
| cis-11-Eicosensäure | Gondoinsäure | 20:1(n-9) |
| cis-13-Docosensäure | Erucinsäure | 22:1(n-9) |
| cis-15-Tetracosensäure | Nervonsäure | 24:1(n-9) |
| t9-Octadecensäure | Elaidinsäure | |
| t11-Octadecensäure | t-Vaccensäure | |
| t3-Hexadecensäure | | trans-16:1 n-13 |

Tabelle 2: Polyungesättigte Fettsäuren

| Systematischer Name | Trivialname | Kurzform |
|---|---|---|
| 9,12-Octadecadiensäure | Linolsäure | 18:2(n-6) |
| 6,9,12-Octadecatriensäure | $\gamma$-Linolsäure | 18:3(n-6) |
| 8,11,14-Eicosatriensäure | Dihomo-$\gamma$-linolensäure | 20:3(n-6) |
| 5,8,11,14-Eicosatetraensäure | Arachidonsäure | 20:4(n-6) |
| 7,10,13,16-Docosatetraensäure | - | 22:4(n-6) |
| 4,7,10,13,16-Docosapentaensäure | - | 22:5(n-6) |
| 9,12,15-Octadecatriensäure | $\alpha$-Linolensäure | 18:3(n-3) |
| 6,9,12,15-Octadecatetraensäure | Stearidonsäure | 18:4(n-3) |
| 8,11,14,17-Eicosatetraensäure | - | 20:4(n-3) |
| 5,8,11,14,17-Eicosapentaensäure | EPA | 20:5(n-3) |
| 7,10,13,16,19-Docosapentaensäure | DPA | 22:5(n-3) |
| 4,7,10,13,16,19-Docosahexaensäure | DHA | 22:6(n-3) |
| 5,8,11-Eicosatriensäure | Meadsäure | 20:3(n-9) |
| 9c 11t 13t Eleostearinsäure | | |
| 8t 10t 12c Calendinsäure | | |
| 9c 11t 13c Catalpicsäure | | |
| 4, 7, 9, 11, 13, 16, 19 Docosaheptadecansäure | Stellaheptaensäure | |
| | Taxolsäure | all-cis-5,9-18:2 |
| | Pinolensäure | all-cis-5,9,12-18:3 |
| | Sciadonsäure | all-cis-5,11,14-20:3 |

Tabelle 3: Acetylenische Fettsäuren

| Systematischer Name | Trivialname |
|---|---|
| 6-Octadecinsäure | Taririnsäure |
| t11-Octadecen-9-insäure | Santalbin- oder Ximeninsäure |
| 9-Octadecinsäure | Stearolinsäure |

(fortgesetzt)

| Systematischer Name | Trivialname |
|---|---|
| 6-Octadecen-9-insäure | 6,9-Octadeceninsäure~ |
| t10-Heptadecen-8-insäure | Pyrulinsäure |
| 9-Octadecen-12-insäure | Crepenynsäure |
| t7,t11-Octadecadiene-9-insäure | Heisterinsäure |
| t8,t10-Octadecadiene-12-insäure | - |
| 5,8,11,14-Eicosatetrainsäure | ETYA |

Tabelle 4: gesättigte Fettsäuren

| Systematischer Name | Trivialname | Kurzform |
|---|---|---|
| Dodecansäure | Laurinsäure | 12:0 |
| Tetradecansäure | Myristinsäure | 14:0 |
| Hexadecansäure | Palmitinsäure | 16:0 |
| Heptadecansäure | Margarinsäure | 17:0 |
| Octadecansäure | Stearinsäure | 18:0 |
| Eicosansäure | Arachinsäure | 20:0 |
| Docosansäure | Behensäure | 22:0 |
| Tetracosansäure | Lignocerinsäure | 24:0 |

Des weiteren sind die Ester der in den Tabellen 1 - 4 aufgeführten Fettsäuren und insbesondere deren Ethylester und Gemische enthaltend diese Fettsäuren und/oder Fettsäureester bevorzugt. Weitere bevorzugte Fettsäuren sind 6,8-Dithianoctansäure, $\gamma$-Linolensäure und $\alpha$-Liponsäure sowie deren Ester.

Des weiteren können als Zusatzstoffe in der einzubringenden Zusammensetzung auch Kontrastmittel enthalten sein.

Als Matrix zur Aufnahme des Wirkstoffs in den Falten des Katheterballons haben sich niedermolekulare Verbindungen und insbesondere Kontrastmittel, Kontrastmittelanaloga oder Kontrastmittel-ähnliche Verbindungen als geeignet herausgestellt. Als Kontrastmittelanaloga oder Kontrastmittel-ähnliche Verbindungen werden Stoffe bezeichnet, welche nicht mit der Bezeichnung "Kontrastmittel" betitelt werden, jedoch die Eigenschaften eines Kontrastmittels besitzen, nämlich durch bildgebende Verfahren und Diagnoseverfahren dargestellt zu werden. Bei diesen Verbindungen handelt es sich zumeist um Stoffe, welche Barium, Iod, Mangan, Eisen, Lanthan, Cer, Praseodym, Neodym, Samarium, Europium, Gadolinium, Terbium, Dysprosium, Holmium, Erbium, Thulium, Ytterbium und/oder Lutetium enthalten.

Als Kontrastmittel können übliche Kontrastmittel für Röntgenaufnahmen, Computertomographie (CT), Kernspintomographie oder Magnetresonanztomographie (MRT) eingesetzt werden.

Prinzipiell zu unterscheiden sind Kontrastmittel, die bei Röntgenuntersuchungen zum Einsatz kommen (Röntgenkontrastmittel), und die, die bei magnetresonanztomographischen Untersuchungen eingesetzt werden (MR-Kontrastmittel), wobei die Röntgenkontrastmittel wie z.B. Jod-Lipiodol® bevorzugt sind.

Im Falle von Röntgenkontrastmitteln handelt es sich um Substanzen, die entweder zu einer vermehrten Absorption einfallender Röntgenstrahlen gegenüber der umgebenden Struktur führen (sog. positive Kontrastmittel) oder einfallende Röntgenstrahlen vermehrt ungehindert durchlassen (sog. negative Kontrastmittel).

Des weiteren sind iodhaltige Kontrastmittel bevorzugt, welche bei der Gefäßdarstellung (Angiographie und Phlebographie) und bei der CT (Computertomographie) verwendet werden.

Insbesondere bevorzugt sind Kontrastmittel mit einem 1,3,5-Triiodbenzolkern, nephrotrope niederosmolare Röntgenkontrastmittel, Amidotrizoesäure, Iothalaminsäure, Iotrolan, Iopamidol, Iodoxaminsäure, Diatrizoesäure, Iomeprol, Iopromid, Desmethoxyacetyl-Iopromid (DAMI) oder 5-Amino-2,4,6-triiodphthalsäure-(2,3-dihydroxypropyl)-amid (ATH).

Im folgenden sind einige Strukturen der insbesondere bevorzugten Kontrastmittel gezeigt:

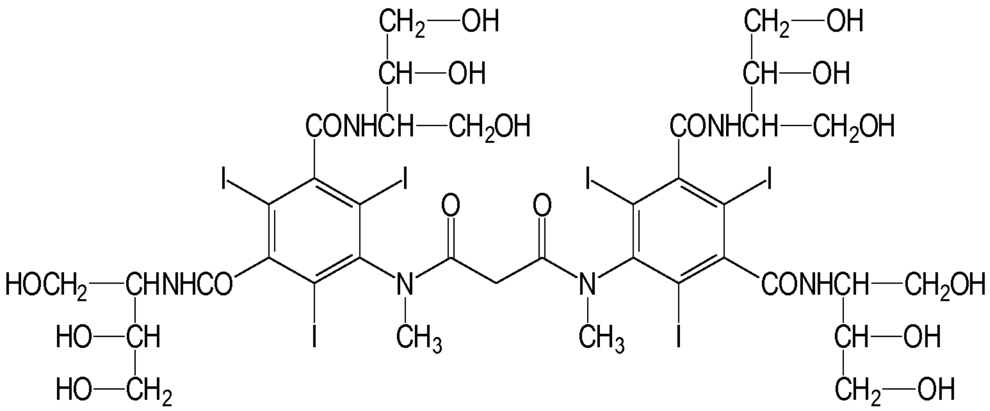

Iotrolan

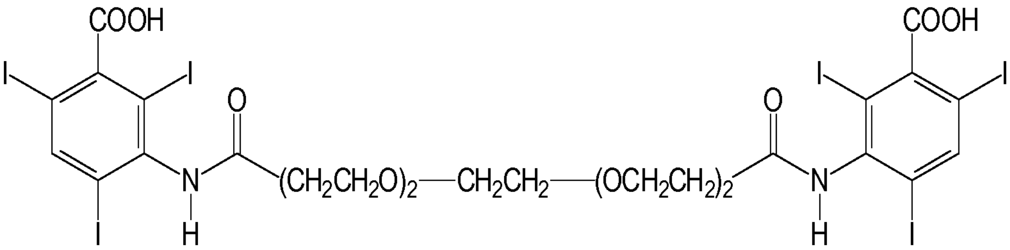

Iodoxaminsäure

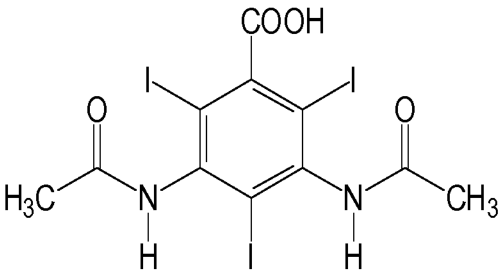

Amidotrizoesäure

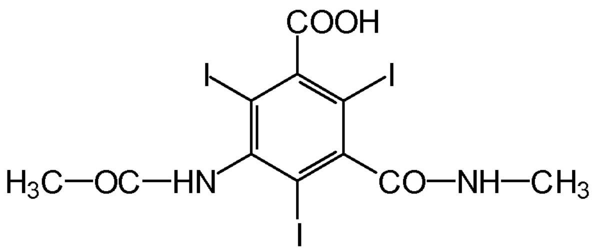

Iothalaminsäure

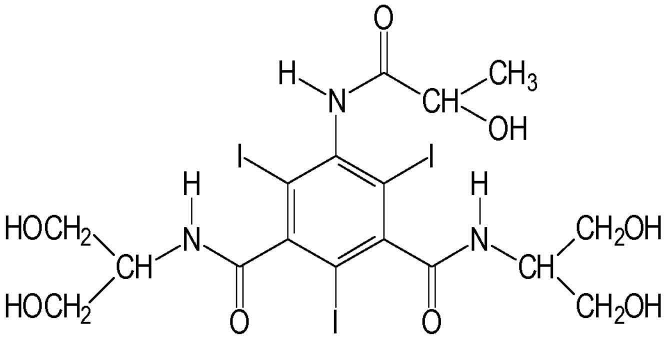

Iopamidol

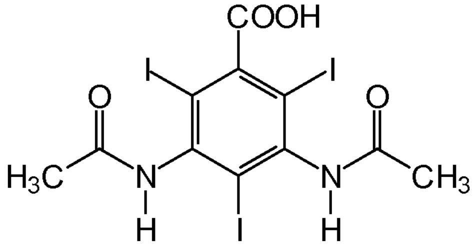

Diatrizoesäure

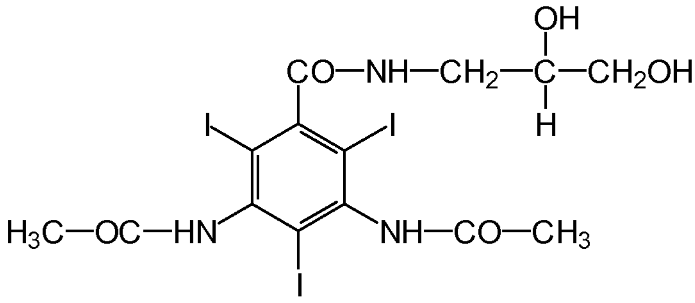

Iomeprol

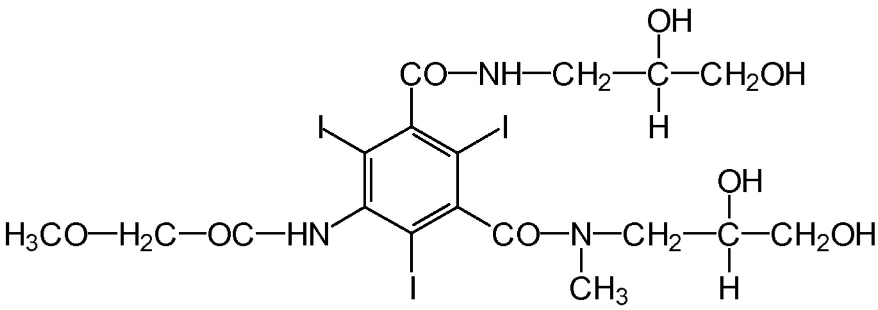

Iopromid

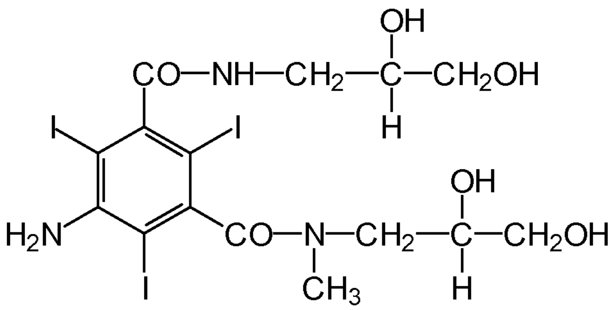

Desmethoxyacetyl-Iopromid (DAMI)

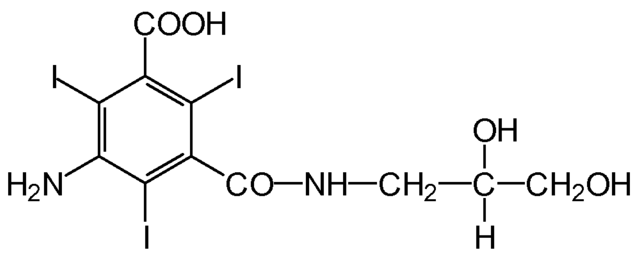

5-Amino-2,4,6-triiodphthalsäure-(2,3-dihydroxypropyl)-amid (ATH)

Eine weitere Klasse von bevorzugten Kontrastmitteln stellen die paramagnetischen Kontrastmittel dar, welche zumeist ein Lanthanoid enthalten.

Zu den paramagnetischen Substanzen, die über ungepaarte Elektronen verfügen, zählt z.B. das Gadolinium ($Gd^{3+}$), das insgesamt sieben ungepaarte Elektronen besitzt. Des weiteren gehören zu dieser Gruppe das Europium ($Eu^{2+}$, $Eu^{3+}$), Dysprosium ($Dy^{3+}$) und Holmium ($Ho^{3+}$). Diese Lanthanoide können auch in chelatisierter Form unter Verwendung von beispielsweise Hämoglobin, Chlorophyll, Polyazasäuren, Polycarbonsäuren und insbesondere EDTA, DTPA sowie DOTA als Chelatbildner eingesetzt werden. Beispiele für Gadolinium-haltige Kontrastmittel sind Gadolinium-Diethylentriaminpentaessigsäure, Gadopentetsäure (GaDPTA), Gadodiamid, Meglumin-Gadoterat oder Gadoteridol

Erfindungsgemäß können natürlich auch Mischungen zweier oder mehrerer Kontrastmittel eingesetzt werden.

Des weiteren können auch physiologisch verträgliche Salze in der Zusammensetzung des Wirkstoffs oder des Wirkstoffgemisches anwesend sein. Bevorzugt sind Lösungen oder Dispersionen eines Wirkstoffs vorzugsweise Paclitaxel oder Rapamycin und insbesondere Paclitaxel zusammen mit einem oder mehreren physiologisch verträglichen Salzen.

Als Salze können bevorzugt Verbindungen enthaltend Natriumkationen, Calcium-, Magnesium-, Zink-, Eisen- oder Lithiumkationen zusammen mit Sulfat-, Chlorid-, Bromit-, Iodid-, Phosphat-, Nitrat-, Citrat- oder Acetatanionen eingesetzt werden. Als Salze können zudem auch ionische Kontrastmittel eingesetzt werden oder ionische Kontrastmittel können den oben genannten Salzen zugesetzt werden.

Einer solchen Lösung, Dispersion oder Aufschlämmung wird der Wirkstoff oder die Wirkstoffkombination zugesetzt. Als Lösungsmittel dient vorzugsweise Wasser eventuell mit Cosolventien. Der Salzgehalt sollte relativ hoch liegen. Bei derartigen Salzlösungen ist das hauptsächliche Lösungsmittel Wasser, welches bis zu 30 Gew.-%, vorzugsweise 20 Gew.-% und insbesondere bevorzugt bis maximal 10 Gew.-% noch ein oder mehrere Cosolventien enthalten kann.

Derartige Salzlösungen werden zumeist für das Spritzverfahren verwendet.

Diese Salzlösung mit Wirkstoff wird unter die Falten des Faltenballon gespritzt und getrocknet. Die Salzbeschichtung ist sehr hygroskopisch und besitzt daher eine hohe Affinität zum Gefäßgewebe. Bei der Dilatation öffnen sich die Falten und drücken die salzige Beschichtung gegen die Gefäßwand. Die Salzbeschichtung klebt dann regelrecht an der Gefäßwand, wo sie mehrere Aufgaben erfüllt. Zum einen führt die lokale sehr hohe Salzkonzentration zu einem hohen isotonischen Druck, der Zellen zum Platzen bringt und zum anderen löst die hohe Salzkonzentration auch harte Plaque und andere Ablagerungen im Gefäß auf und setzt zudem noch den Wirkstoff frei, welcher insbesondere die Proliferation der glatten Muskelzellen unterbindet.

Nach wenigen Minuten bis maximal ein paar Stunden ist je nach Menge die auf die Gefäßwand übertragene Salzbeschichtung vollständig aufgelöst.

Anstelle oder in Kombination mit den vorgenannten Salzen können auch Aminosäuren, Oligopeptide, Polyaminosäuren, Peptide und/oder Vitamine eingesetzt werden.

Geeignete Aminosäuren sind: Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Prolin, 4-Hydroxyprolin, N,N,N-Trimethyllysin, 3-Methylhistidin, 5-Hydroxylysin, O-Phosphoserin, γ-Carboxygiutamat, ε-N-Acetyllysin, ω-N-Methylarginin, Citrullin, Ornithin und Derivate dieser Aminosäuren.

Geeignete Vitamine umfassen: Vitamin A, Vitamin C (Ascorbinsäure), Vitamin D, Vitamin H, Vitamin K, Vitamin E, VitaminB1, VitaminB2, VitaminB3, VitaminB5, VitaminB6, VitaminB12, Thiamin, Riboflavin, Niacin, Pyridoxin und Folsäure.

Ferner sind auch liposomale Formulierungen des Wirkstoffs oder Wirkstoffgemisches für die Beschichtung bzw. Befüllung von Katheterballons einsetztbar.

Die liposomalen Formulierungen werden vorzugsweise hergestellt, indem in einem ersten Schritt der Wirkstoff (z.B. Paclitaxel oder Rapamycin) bzw. die Wirkstoffkombination in einem wässrigen Medium oder Puffermedium gelöst und anschließend mit Lösungen, die membranbildende Substanzen enthalten, in Kontakt gebracht wird. Bei diesem Verfahren ergeben sich hohe Einschlussraten von mindestens 30% bis zu 95%.

Membranbildende Substanzen sind geladene amphiphile Verbindungen bevorzugt Alkylcarbonsäuren, Alkylsulfonsäuren, Alkylamine, Alkylammoniumsalze, Phosphorsäureester mit Alkoholen, natürliche sowie synthetische Lipide, wie Phosphatidylglycerol (PG), Phosphatidylserin (PS), Derivate des Phophatidylethanolamins (PE-Derivate) sowie des Cholesterols, Phosphatidsäure, Phosphatidylinositol, Cardiolipin, Sphingomyelin, Ceramid in seinen natürlichen, halb-synthetischen oder synthetischen Formen, Stearylamin und Stearinsäure, Palmitoyl-D-glucuronid und/oder geladene Sphingolipide, wie z. B. Sulfatid.

Als neutrale membranbildende Substanzen fungieren an sich bekannte Komponenten wie z. B. Phosphatidylcholin (PC), Phophatidylethanolamin (PE), Steroide, vorzugsweise Cholesterol, komplexe Lipide und/oder neutrale Sphingolipide.

Die Gewinnung der Liposomen aus der wässrigen Lösung erfolgt ebenfalls nach an sich bekannten Techniken so z. B. durch Dialyse, Ultrafiltration, Gelfiltration, Sedimentation oder Flotation. Die Liposomen besitzen einen durchschnittlichen Durchmesser von 10 bis 400 nm.

Derartige liposomale Formulierungen lassen sich auch bevorzugt in die Falten eines Faltenballons mittels Spritzverfahren oder Kapillarverfahren (Pipettierverfahren) einbringen.

Das erfindungsgemäße Faltenbeschichtungsverfahren oder Faltenbefüllungsverfahren ist das Spritzverfahren.

Somit betrifft die vorliegende Erfindung ein Verfahren zur Beschichtung oder Befüllung der Falten eines Katheterfaltenballons auf folgende Weise:

eine Spritze, welche einen kontinuierlichen Fluß einer wirkstoffenthaltenden Zusammensetzung abgibt, wird relativ zum Katheterfaltenballon entlang der Falte bewegt.

Vorteilhaft ist, dass dieses Beschichtungs- oder Befüllungsverfahren vorzugsweise im komprimierten oder deflatierten Zustand oder maximal 10% inflatierten Zustand des Katheterballons durchgeführt wird. Unter dem Begriff "10% inflatierten Zustand" soll verstanden werden, dass der Katheterballon 10% der Inflation, d.h. der Aufdehnung bis zur bei der Dilatation geplanten Maximalausdehnung erfahren hat. Wird die bei der Dilatation vorgesehene Aufdehnung mit 100% bezeichnet und der deflatierte Zustand mit 0% angesetzt, so ergibt sich eine 10%ige Inflation gemäß folgender Formel:

$$\text{(Durchmesser des Katheterballons im deflatierten Zustand)} + \text{(Durchmesser des Katheterballons im inflatierten Zustand – Durchmesser des Katheterballons im deflatierten Zustand)} / 10$$

Ferner können gemäß dem erfindungsgemäßen Verfahren mehrere oder alle Falten gleichzeitig beschichtet oder befüllt werden und die Beschichtung oder Befüllung kann gezielt erfolgen. Eine gezielte Befüllung der Falten oder gezielte Beschichtung der Falten soll heißen, dass nur die Falten befüllt oder beschichtet werden und die Oberfläche des Katheterfaltenballons außerhalb der Falten nicht beschichtet wird.

Eine bevorzugt verwendete Zusammensetzung aus Wirkstoff, Lösungsmittel und Kontrastmittel hat die Konsistenz einer Paste oder einer dickflüssigen Masse oder einer viskosen Dispersion oder Emulsion oder eines zähen Breis.

Diese Zusammensetzung hat den Vorteil, dass sie nicht polymerisiert und während des Beschichtungsvorgangs ihre Konsistenz beibehält. Diese Paste oder (hoch)viskose Masse oder dickflüssige Aufschlämmung wird mittels einer Spritzvorrichtung, vorzugsweise einer Düse wie in Fig. 1 gezeigt unter Druck in die Falten eingebracht.

Die Düse kann sofern nötig, die Ballonfalten aufweiten und gezielt die durch die Falten gebildeten Hohlräume befüllen. Die Faltenballons weisen in der Regel 4 oder mehr Falten auf, welche einzeln befüllt werden.

Als besonders vorteilhaft hat sich erwiesen, nach Befüllung einer oder mehrerer oder aller Falten den Faltenballon in Richtung der Öffnungen der Falten zu rotieren. Diese Drehung bewirkt, dass die dickflüssige Paste vollständig und gleichmäßig in den Falten verteilt wird und eventuelle Lufteinschlüsse freigesetzt werden. Die Rotationsrichtung ist in Fig. 2 angedeutet. Nach der Drehung des Faltenballons kann eine weitere Befüllung von bereits befüllten oder leeren Falten erfolgen.

Während und/oder nach der Rotation trocknet die Zusammensetzung in den Falten entweder bei Atmosphärendruck oder unter vermindertem Druck. Trocknung oder Aushärtung der Zusammensetzung erfolgt durch die Entfernung des mindestens einen Alkohols durch Verdunstung. Die getrocknete Zusammensetzung hat eine poröse Konsistenz und löst sich sehr leicht von der Ballonoberfläche bei der Dilatation ab. Der Alkohol als Lösungsmittel wurde bis auf die üblichen Rückstände entfernt und das Kontrastmittel bildet eine poröse Matrix für den Wirkstoff und ist zudem in der Lage, nach Dilatation des Faltenballons den Wirkstoff schnell und in großer Konzentration freizusetzen. Zudem hat das erfindungsgemäße Verfahren den Vorteil, sehr materialschonend zu arbeiten, da nur die Falten beschichtet oder befüllt werden können und somit sich kein Wirkstoff auf der äußeren Ballonoberfläche befindet, der bei der Einführung des Katheters verloren geht.

Im Folgenden wird auf das erfindungsgemäße Beschichtungs- und Befüllungsverfahren genauer eingegangen.

Spritzverfahren oder Spritzenverfahren:

Bei dem erfindungsgemäßen Verfahren wird eine feine Spritze oder Nadel oder Düse am proximalen oder distalen Ende einer Falte angesetzt und diese Abgabevorrichtung in Form einer Spritze, Nadel oder Düse entlang der Längsrichtung der Falte relativ zur Falte bewegt und pro zurückgelegter Teilstrecke eine bestimmte Menge einer wirkstoffenthaltenden Zusammensetzung abgegeben.

Der Katheterballon und die Abgabevorrichtung bewegen sich beide relativ zueinander. Eine Bewegung auf einer Geraden in entgegengesetzter Richtung ist bevorzugt.

Von der Abgabevorrichtung, d.h. der Spritze, Nadel oder Düse oder ähnlichem wird vorzugsweise eine mittel- bis dickviskose wirkstoffenthaltende Zusammensetzung vorzugsweise in Form einer Paste oder eines Gels oder eines Öls in das Falteninnere abgegeben. Die Viskositäten bevorzugter Lösungen liegen zwischen $10^1$ bis $10^6$ mPa·s, vorzugsweise zwischen $10^2$ bis $10^5$ mPa·s und insbesondere bevorzugt zwischen $10^3$ bis $10^4$ mPa·s.

Somit eignen sich als wirkstoffenthaltende Zusammensetzungen insbesondere solche mit den oben bezeichneten Ölen, Alkoholen (insbesondere Diolen und Polyolen), Fettsäuren, Fettsäureestern, Aminosäuren, Polyaminosäuren, membranbildenden Substanzen, liposomalen Formulierungen und/oder Salzen.

Bei dem Beschichtungsvorgang reicht die Spitze der Spritze, Düse oder Nadel bis ca. in die Mitte des Falteninneren, also bis ca. in die Mitte der Falte. Dort wird ein kontinuierlicher Fluß der wirkstoffenthaltenden Zusammensetzung derart abgegeben, dass Abgabegeschwindigkeit und Abgabemenge in Bezug auf die relative Bewegungsgeschwindigkeit von Abgabevorrichtung und Katheterballon geeignet sind, die Falte bzw. das Falteninnere zu mindestens 50 Vol.-%, bevprzugt zu mindestens 70 Vol.-% und insbesondere bevorzugt zu mindestens 85 Vol.-% mit der wirkstoffenthaltenden Zusammensetzung zu füllen.

Die Befüllung einer Falte dauert ca. von 5 bis 80 Sekunden, vorzugsweise ca. 15 bis 60 Sekunden und insbesondere bevorzugt ca. 25 bis 45 Sekunden bei einer Faltenlänge von 10 mm.

Der Katheterballon befindet sich beim Befüllungsvorgang der Falten im komprimierten, d.h. deflatierten Zustand. Eine auch nur teilweise oder geringfügige Inflation des Katheterballons ist in der Regel nicht erforderlich, um die Falten ein wenig zu öffnen. Dennoch kann die Befüllung der Falten bei einer geringfügigen Inflation des Katheterballons von maximal 10% des bei der Dilatation vorgesehenen Durchmessers erfolgen. Die Befüllung der Falten kann zudem bei einer leichten Aufweitung der Falten erfolgen indem 100 kPa (1 bar) Überdruck, vorzugsweise 50 kPa (0,5 bar) Überdruck angelegt werden, um die Falten leicht aufzuweiten.

Dieses Beschichtungsverfahren kann natürlich auch mit dünnflüssigen wirkstoffenthaltenden Zusammensetzungen durchgeführt werden ist aber eher für ölige Zusammensetzungen geeignet als auch für hochkonzentrierte Salzlösungen.

Ferner bietet dieses Verfahren noch den Vorteil, dass mehr als eine Falte und insbesondere alle Falten gleichzeitig beschichtet oder befüllt werden können. Dabei wird eine ringförmige Anordnung von Abgabevorrichtungen entsprechend der Anzahl an Falten derart angeordnet, dass pro Falte ein Abgabevorrichtung bereitsteht. Durch eine geringfügige Drehung werden die Spitzen der Abgabevorrichtungen in die Falten eingeführt und ca. mittig im Falteninneren platziert. Durch eine relative und gleichzeitige Bewegung der Abgabevorrichtungen relativ zur Längsrichtung der Falten, können alle Falten gleichzeitig mit einem kontinuierlichen und gleichmäßigen Fluß an wirkstoffenthaltender Zusammensetzung befüllt werden.

Beim Beschichten oder Befüllen einer oder aller Falten kann sich der Katheterballon in senkrechter oder horizontaler Lage oder einer schrägen Lage befinden.

Wurden in der wirkstoffenthaltenden Zusammensetzung flüchtige Lösungsmittel verwendet, so kann eine Trocknung des Falteninhalts erforderlich sein. Bei flüchtigen Lösungsmitteln erfolgt dies bevorzugt zuerst aufgrund Verdunstung des oder der flüchtigen Lösungsmittel.

Danach kann eine Endtrocknung erfolgen, wobei der Katheterballon in Richtung der Faltenöffnungen gesehen vom Falterinnenraum gedreht wird. Auf dieses Verfahren wird weiter unten ausführlich eingegangen.

Die Drehung oder Rotation des Katheterballons in Richtung der Faltenöffnungen kann zudem auch dazu dienen, die in den Falten bzw. unter den Falten befindlichen Zusammensetzungen gleichmäßig in der jeweiligen Falte zu verteilen.

Diese Rotation des Faltenballons kann insbesondere bei der Verwendung von öligen oder pastenförmigen wirkstoffenthaltenden Zusammensetzungen vorteilhaft sein, um eine gleichmäßige Verteilung der wirkstoffenthaltenden Zusammensetzung in den Falten als auch auf der Innenoberfläche der Falten zu gewährleisten.

Wie hierin verwendet bezieht sich der Begriff "Beschichtung" daher auch vorrangig auf die Beschichtung der Falteninnenoberflächen, wobei der gesamte Falteninnenraum in der Regel nicht mit wirkstoffenthaltender Zusammensetzung bzw. nach der Trocknung mit der verbliebenen Zusammensetzung ausgefüllt ist. Hingegen bezeichnet der Begriff "Befüllung" eher die vollständige Ausfüllung des Falteninnenraumes mit wirkstoffenthaltender Zusammensetzung.

Werden Lösungsmittel verwendet, welche durch Trocknung entfernt werden, so ist eine Befüllung in der Regel nicht zu erreichen und es wird eher von Beschichtung der inneren Oberflächen der Falten gesprochen.

Werden hingegen als Trägerstoffe oder Hilfsstoffe Substanzen mit hohen Siedepunkten wie beispielsweise Öle verwendet, so ist auch eine mehr oder weniger vollständige Befüllung der Falten möglich, sofern keine nennenswerten Mengen von flüchtigen Substanzen in der wirkstoffenthaltenden Zusammensetzung anwesend sind.

Dieses Spritzverfahren oder Spritzenverfahren eignet sich insbesondere zur Einbringung von wirkstoffenthaltenden Zusammensetzungen in die Falten von Katheterfaltenballons, welche mittels herkömmlicher Tauch- oder Sprühverfahren nicht auf einen Katheterballon aufgetragen geschweige denn in die Falten eingebracht werden können.

Im Gegensatz zu den herkömmlich verwendeten festen Beschichtungen auf Stents oder auf Katheterballons bieten die öligen und pastenförmigen Beschichtungen und Befüllungen den Vorteil, dass diese wirkstoffenthaltenden Zusammensetzungen eben nicht vollständig trocknen, sondern ihre Konsistenz weitgehend beibehalten.

Wird am Ort der stenosierten Stelle der Katheterballon inflatiert bzw. dilatiert, so wird diese ölige oder pastenförmige Zusammensetzung zumindest teilweise auf die Gefäßwand übertragen und dient als Wirkstoffreservoir für eine verzögerte Wirkstoffabgabe von mehreren Stunden bis Tagen an das umliegende Gewebe und hat zudem die positive Eigenschaft Plaque aufzulösen und wird selber danach biologisch abgebaut, ohne physiologisch bedenkliche Abbauprodukte freizusetzen.

Rotationstrocknung:

Wie oben erwähnt können die beschichteten oder befüllten Katheterballons nach dem Befüllen oder Beschichten

jeder Falte oder nach der Beschichtung oder Befüllung aller Falten oder der zu beschichtenden bzw. zu befüllenden Falten, falls nicht alle Falten beschichtet oder befüllt werden sollen, im rotierenden Zustand getrocknet werden.

Diese Rotationstrocknung hat mehrere Vorteile. Zum einen wird dadurch die wirkstoffenthaltende Zusammensetzung getrocknet und zudem gleichmäßig in den Falten als auch auf der Oberfläche innerhalb der Falten verteilt.

Die Rotationstrocknung ist insbesondere bei öligen oder zähflüssigen wirkstoffenthaltenden Zusammensetzung geeignet, eine gleichmäßige Verteilung der Zusammensetzung in der jeweiligen Falte zu erreichen.

Zudem kann bei der Rotation des Katheterballons Vakuum angelegt werden, um eine intensive Trocknung der wirkstoffenthaltenden Zusammensetzung zu erreichen.

Bei der Trocknung im Vakuum treten gerade bei zähflüssigen, hochviskosen oder in den festen Zustand übergehenden Lösungen Siedeverzüge auf, d.h. in dem Öl oder Feststoff eingeschlossene Lösungsmittelreste werden spontan freigesetzt und zerreißen oder sprengen die Beschichtung oder Befüllung. Durch eine Trocknung im Vakuum bei gleichzeitiger Rotation wird werden diese Siedeverzüge vermieden und es wird eine getrocknete gleichmäßige Beschichtung der inneren Oberfläche der Falten erhalten.

Zudem ist die Drehrichtung der Rotation entscheidend. Die Drehrichtung erfolgt in Richtung der Faltenöffnungen, wenn man dies aus dem Inneren der Falte betrachtet. In Figur 2 ist die Drehrichtung angezeigt und der Katheterballon wird so wie ein Schaufelrad einer Schaufelradbaggers gedreht, damit die wirkstoffenthaltende Zusammensetzung aufgrund der Rotationskraft in das Falteninnere gedrückt wird.

Vorzugsweise wird der Faltenballon mit einer Rotationsgeschwindigkeit von 50 bis 500, vorzugsweise 150 bis 300 Umdrehungen pro Minute gedreht.

Das Spritzverfahren ist besonders gut für mittelviskose, viskose bis hochviskose Zusammensetzungen einsetzbar.

Der Begriff Viskosizität bezieht sich auf die dynamische Viskosität [η]:

$$[\eta] = \frac{\mathrm{kg}}{\mathrm{m} \cdot \mathrm{s}} = \mathrm{Pa} \cdot \mathrm{s} = \frac{\mathrm{Ns}}{\mathrm{m}^2}$$

Das Spritzverfahren kann vorzugsweise bei dickviskosen Zusammensetzungen eingesetzt werden. Bevorzugt sind Viskositäten bei Raumtemperatur im Bereich von Ölen (Olivenöl: $10^2$ mPa s), Honig ($10^3$ mPa s), Glycerin (1480 mPa s) oder Sirup ($10^5$ mPa s). Selbstverständlich funktioniert dieses Verfahren auch mit dünnviskosen Lösungen mit $\eta \leq 10^2$ mPa s.

Überraschenderweise wurde ferner gefunden, dass eine Zusammensetzung umfassend mindestens ein Kontrastmittel und mindestens einen antiproliferativen, antiinflammatorischen, antiphlogistischen, zytostatischen, zytotoxischen, antiangiogenen, anti-restenotischen oder antithrombotischen Wirkstoff und mindestens einen Alkohol als Lösungsmittel sich für die Beschichtung bzw. Befüllung der Falten von Katheterfaltenballons besonders gut eignet.

Es wurde bewußt kein Polymer als Wirkstoffträger eingesetzt, welches durch das erfindungsgemäße Beschichtungsverfahren nur schwer in die Falten des Katheterballons einzubringen war, die Falten teilweise verklebte, so dass bei der Dilatation des Katheterfaltenballons eine ungleichmäßige Öffnung der Falten und eine nicht einheitliche Wirkstoffabgabe eintrat.

Beim Spritzenverfahren wird zur Befüllung der Falten eines Faltenballons vorzugsweise eine feine Düse oder Kanüle unter die Falte geschoben und die einzubringende Mischung wird in die Falte gespritzt, wobei vorzugsweise die Düse oder Kanüle entlang der Falte bewegt wird oder bei ortfester Düse oder Kanüle (nicht erfindungsgemäß) der Faltenballon in Längsrichtung der Falte bewegt wird. Dieses Verfahren ermöglicht eine sehr präzise und genaue Beschichtung einer jeden einzelnen Falte bzw. des gesamten Faltenballons. Ein eventuell verwendetes Lösungsmittel verdunstet oder wird im Vakuum entfernt.

Hat die einzubringende Mischung oder Lösung eine Konsistenz, so dass sie in die Falten hineinfließen kann, dann wird der Faltenballon mit einer Falte noch oben horizontal gelagert oder vorzugsweise um 5 bis 25 Grad geneigt, so dass die Spritze oder Düse am unteren Ende des Faltenballons an der Faltenöffnung angesetzt werden kann und die Mischung eigenständig in die Falte hineinfließt und diese voll ausfüllt.

Bei diesen Salzlösungen mit hohem Salzgehalt wird vorzugsweise Wasser als Lösungsmittel eingesetzt, weil Wasser das Ballonmaterial nicht angreift und beschädigt. Sobald die Mischung eine Konsistenz hat, dass sie nicht mehr aus der Falte herausfließen kann, wird der Faltenballon gedreht und die nächste Falte wird befüllt bis in der Regel alle 4 bis 6 Falten des Ballons befüllt sind. Faltenballons werden bevorzugt im komprimierten Zustand beschichtet, wobei einige spezielle Ausführungsformen von Faltenballons auch im expandierten Zustand beschichtet werden können.

Ein solches Beschichtungsverfahren umfasst die Schritte

a) Bereitstellen eines Faltenballons

b) Platzierung einer Falte des Ballons in einer horizontalen oder bis zu 25 Grad geneigten Position,
c) Ansetzen der Spritzenöffnung an die dem Kopfende des Ballons zugewandten Faltenöffnung,
d) Ausführung einer Relativbewegung von Spritzenöffnung und Faltenballon in Längsrichtung der Falte,
e) Befüllung der Falte während des Bewegungsvorgangs mit einer Mischung aus einem Wirkstoff und einem Salz und/oder einem ionischen Kontrastmittel in einem geeigneten Lösungsmittel,
f) sofern erforderlich Trocknung der in der Falte befindlichen Mischung bis zu einem Grad, der ein Auslaufen der Mischung aus der Falte verhindert,
g) Drehung des Ballons um 360° dividiert durch die Anzahl der Falten,
h) Wiederholung der Schritte b) bis g) bis alle Falten befüllt sind und
i) Trocknung der Mischungen in des Falten bis sich die Mischung verfestigt.

Werden dünnflüssigere Mischungen eingesetzt, so wird bei Schritt c) die Spritzenöffnung an das Fußende angesetzt und ohne Relativbewegung gemäß Schritt d) die Falte vorwiegend aufgrund von Kapillarkräften befüllt (nicht erfindungsgemäß).

## Figurenbeschreibung

Fig. 1 zeigt einen Katheterfaltenballon mit vier Falten im nicht dilatierten Zustand, wobei eine Falte gerade mittels einer Spritzdüse mit einer Zusammensetzung aus Kontrastmittel, Wirkstoff und Lösungsmittel befüllt wird.

Fig. 2 zeigt einen vollständig in allen vier Falten befüllten Katheterfaltenballon im nicht expandierten Zustand, wobei die in den Falten befindliche Zusammensetzung während der Rotation des Faltenballons getrocknet bzw. das Lösungsmittel entfernt wird.

## Beispiele

### Beispiel 1:

Ein üblicher Katheterfaltenballon mit vier Falten wird im zusammengefalteten Zustand mit einer Zusammensetzung aus Iopromid, welches als Lösung unter der Marke Ultravist® kommerziell erhältlich ist, Paclitaxel, Glycerin und Ethanol mittels einer Spritzdüse befüllt.

Die Zusammensetzung enthält 0,150 g Iopromid, 0,300 g Paclitaxel, 0,220 g Glycerin und 1,130 g Ethanol.

In jede Falte wird ca. 174 μg - 621 μg der Zusammensetzung eingebracht.

Nach Befüllung aller Falten wird der Faltenballon für 1 Minute in Richtung der Faltenöffnungen rotiert und über Nacht getrocknet.

Insgesamt wurden 696 μg - 2484 μg der getrockneten Zusammensetzung verteilt in vier Falten eingebracht.

### Beispiel 2 (nicht erfindungsgemäß):

Ca. 10mg $KMnO_4$ werden in 500μl Wasser gelöst und soviel PVP wie möglich zugegeben. Die Masse wird flächig auf einer Polypropylenunterlage ausgebreitet und bei Raumtemperatur über Nacht trocknen gelassen.
Von dieser spröden Masse werden 4,5 mg in 0,5 ml Chloroform oder 0,6 ml Methanol oder 0,8 ml DMSO gelöst. Ungelöste Partikel können abfiltriert werden. Zu dieser Lösung werden 100 μg Paclitaxel gegeben und nach Zugabe von 12,0 μl Leinöl wird die resultierende Lösung mittels des Faltensprühverfahrens über 4 in Reihe befindliche Düsen mit einem Abstand von 2 mm zueinander gleichmäßig in eine Falte gesprüht.

Nach der Beschichtung der ersten Falte im waagerechten Zustand wird die Beschichtung ohne Vakuum getrocknet und der vier Falten umfassende Ballon wird um 90 Grad gedreht, um die nächste oben liegende Falte zu beschichten.

Nach erfolgter Beschichtung aller Falten erfolgt eine Trocknung im Vakuum unter Rotation des Katheterballons gemäß dem oben beschriebenen Rotationstrocknungsverfahren.

### Beispiel 3 (nicht erfindungsgemäß):

Paclitaxel (250 mg) oder Rapamycin (250 mg) oder eine Kombination aus Paclitaxel (150 mg) und Rapamycin (150 mg) werden in einem wässrigen Medium mit einem Ethanolanteil von ca. 5 Vol.-% und bei einem pH-Wert von 4 bis 5 gelöst oder suspendiert und anschließend mit einer Lösung aus Phosphatidylserin und Phosphatidylcholin im Gewichtsverhältnis 50 : 50 versetzt.

Es bildet sich eine liposomale Formulierung bei der der Wirkstoff oder die Wirkstoffkombination in den Vesikeln eingeschlossen ist.

Die Gewinnung der Liposomen aus der wässrigen Lösung erfolgt mittels Ultrafiltration, wobei eine Gewinnung der Liposomen nicht unbedingt zwingend ist.

Die gewonnenen Liposome oder die liposomale wässrige Formulierung wird in eine Kavität gefüllt und mittels des Pipettierverfahrens in eine Falte gefüllt, wobei eine Kapillare im Winkel von ca. 40 Grad an das distale Ende der Falte angesetzt wird. Der Katheterballon liegt dabei horizontal und die zu beschichtende Falt liegt oben. Aufgrund von Kapillarkräften zieht sich die Zusammensetzung innerhalb von ca. 48 Sekunden in die Falte. Bevor die Zusammensetzung das proximale Ende der Falte erreicht hat, wird die Kapillare entfernt, so dass auch noch die am Ansatzpunkt der Kapillare vorhandene Zusammensetzung in die Falte gezogen wird.

Der beschichtete Katheterballon wird danach an der Luft getrocknet und anschließend mittels des oben beschriebenen Rotationstrocknungsverfahren abschließend getrocknet.

**Beispiel 4:**

400 mg Trapidil werden mit 1 ml Safloröl vermischt und mittels des oben beschriebenen Spritzenverfahrens gleichzeitig in die vier Falten eines Faltenballons eingebracht.

Die Abgabeöffnungen der Abgabevorrichtung werden am distalen Ende der jeweiligen Falte angesetzt und mit einer Geschwindigkeit von 1 cm pro Minute in Richtung des proximalen Endes in der Falte bewegt während ein kontinuierlicher Fluß an öliger Trapidillösung in die Falte abgegeben wird.

Eine Trocknung der beschichteten oder besser gesagt befüllten Falten ist nicht erforderlich. Jede Falte wurde mit ca. 1,5 mg öliger Tripidil-Zusammensetzung befüllt.

**Beispiel 5 (nicht erfindungsgemäß):**

300 mg Rapamycin werden in 1 ml Essigsäureethylester gelöst und mit 200 mg EPA (5,8,11,14,17-Eicosapentaensäure) versetzt. Durch Zugabe von Ethanol kann die Viskosität so eingestellt werden, dass sich eine sprühbare Mischung ergibt.

Mittels des Faltensprühverfahrens werden alle 6 Falten eines Faltenballons gleichzeitig beschichtet. Die Sprühvorrichtung besteht aus einer Anordnung von insgesamt 6 Reihen aus jeweils drei Sprühdüsen. Die Reihen von jeweils drei Sprühdüsen sind auf einem Drehgestellt gelagert und können einzeln in die jeweilige Falte des Faltenballons eingeführt werden.

Sind die Reihen an Sprühdüsen richtig platziert, wird aus jeder Düse die wirkstoffenthaltende Zusammensetzung unter die jeweilige Falte abgegeben.

Der Katheterballon befindet sich in einer vorzugsweise senkrechten Position und die drei Düsen einer Sprühreihe haben einen Abstand von 3 mm zueinander.

Nach erfolgter Beschichtung der Falten, welche auch intervallmäßig durchgeführt werden kann, werden die beschichteten Falten an der Luft bei Normaldruck und bei Raumtemperatur getrocknet und können anschließend bei einer Temperatur von 50°C bis 70°C bei Normaldruck weiter getrocknet werden falls nötig.

Der Faltenballon wurde in den Falten mit insgesamt 76 mg festen Substanzen beschichtet.

**Beispiel 6 (nicht erfindungsgemäß):**

Eine Lösung aus 250 μg Paclitaxel in einem Gemisch aus Ethanol, n-Butanol und Ethylenglykol im Volumenverhältnis 50 : 25 : 25 wird angesetzt und zu dieser Lösung werden 200 μg Vitamin A gegeben.

Diese Zusammensetzung wird dann in eine Abgabevorrichtung mit insgesamt 4 Kanülen gefüllt. Jede dieser vier Kanülen wird im spitzen Winkel von ca. 30 Grad an eine der vier Falten eines Katheterfaltenballons angesetzt und die vier Falten werden gleichzeitig mittels des Pipettierverfahrens befüllt.

Nach der Befüllung wird der Katheterballon langsam in Rotation versetzt und zeitgleich wird Vakuum angelegt und die Rotationsgeschwindigkeit mit steigendem Vakuum kurzzeitig bis auf 1.200 Umdrehungen gesteigert und der Falteninhalt wird gemäß dem oben beschriebenen Rotationstrocknungsverfahren getrocknet.

**Beispiel 7:**

Borretschöl oder Leinöl und Paclitaxel im Gewichtsverhältnis 80 zu 20 werden miteinander vermischt und im Mischungsverhältnis 1:1 in Chloroform oder DMSO gelöst, so dass eine viskose Zusammensetzung entsteht.

Diese Zusammensetzung wird mittels des oben beschriebenen Spritzenverfahrens in eine Falte eines Faltenballons mit insgesamt 5 Falten gefüllt. Dazu wird der Katheterballon fixiert und so gedreht, dass in die zu befüllende

Falte die Nadel der Abgabevorrichtung eingeführt werden kann. Die Nadel wird am proximalen Ende des Katheterballon in die Falte bis ca. zur Mitte eingeführt und während einer Zeit von ca. 50 Sekunden wird die Nadel gleichmäßig in Längsrichtung der Falte bewegt während ein kontinuierlicher Fluß an viskoser wirkstoffenthaltender Zusammensetzung in die Falte abgegeben wird.

Die durchschnittliche Beschichtungsmasse an Zusammensetzung pro Falte kann zwischen 0,4 und 2,8 mg je nach Ausgestaltung der Falte betragen. Im vorliegenden fall beträgt die durchschnittliche Beschichtungsmasse an Zusammensetzung pro Falte ca. 0,8 mg im getrockneten Zustand.

Nach der Befüllung aller 5 Falten wird der Katheterballon unter Verwendung des oben beschriebenen Rotationstrocknungsverfahren getrocknet.

Der Faltenballon ist mit insgesamt ca. 4,0 mg getrockneter Zusammensetzung befüllt bzw. beschichtet worden.

**Beispiel 8 (nicht erfindungsgemäß):**

500μg Paclitaxel wird in 1 ml wasserfreiem Ethanol und 0,4 ml Essigsäure gelöst und mittels des Pipettierverfahrens in die Falten eines Katheterballon gefüllt.

Die anschließende Trocknung erfolgt nach Verdunstung des Lösungsmittels gemäß dem oben beschriebenen Rotationstrocknungsverfahren.

Nach der Beschichtung und Trocknung befinden sich in jeder Falte ca. 10 μg Pacl itaxel.

**Beispiel 9:**

200 μg Paclitaxel werden in 0,5 ml Ethanol und 0,5 ml DMSO und 0,1 ml Essigsäure gelöst. Ferner wird eine Lösung von 350 μg Kaliumacetal in Ethanol - Wasser im Volumenverhältnis 90 : 10 bereitgestellt. Die Kaliumacetatlösung wird nun zur Paclitaxellösung gegeben und die Lösungsmittel können verdunsten, bis die ersten Bestandteile beginnen auszufallen oder die Zusammensetzung beginnt sich zu trüben oder eine mittelviskose Zusammensetzung entsteht.

Diese Zusammensetzung wird mittels dem oben beschriebenen Spritzenverfahren in die Falten des Katheterballons gegeben. Erfindungsgemäß können die Falten einzeln und nacheinander befüllt oder auch gleichzeitig zusammen befüllt werden. Nach Verdunstung des Lösungsmittels erfolgt die finale Trocknung gemäß dem Rotationstrocknungsverfahren wie eingangs beschrieben.

**Beispiel 10a (nicht erfindungsgemäß):**

500 μg Rapamycin und 25 mg lopamidol werden in 1 ml Ethanol gelöst.

Diese Lösung kann in der vorliegenden Form in die Falten des Katheterballons mittels des Faltensprühverfahrens eingebracht werden, wobei die Falten wie oben beschrieben einzeln oder gleichzeitig beschichtet werden können.

Trocknung erfolgt vorzugsweise mittels des oben beschriebenen Rotationstrocknungsverfahrens.

**Beispiel 10b (nicht erfindungsgemäß):**

Die Lösung aus Beispiel 10a wird durch Zugabe von Propylenglykol und/oder Glycerin auf eine Viskosität von $10^3$ bis $10^4$ mPa·s eingestellt.

Die so erhaltene Zusammensetzung wird gemäß dem Pipettierverfahren in die Falten eingebracht werden, wobei die Falten einzeln nacheinander oder gleichzeitig zusammen befüllt werden können.

Die abschließende Trocknung erfolgt vorzugsweise mittels des oben beschriebenen Rotationstrocknungsverfahrens.

**Beispiel 10c:**

Die Lösung aus Beispiel 10a oder aus Beispiel 10b wird durch Zugabe eines Öles ausgewählt aus der Gruppe bestehend aus Leinöl, Flachsöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl auf eine Viskosität größer als $10^4$ mPa·s eingestellt.

Die so erhaltene Zusammensetzung wird mittels Spritzverfahren wie oben beschrieben in die Falten des Katheterballons eingebracht, wobei die Falten einzeln nacheinander oder gleichzeitig zusammen befüllt werden können.

Die abschließende Trocknung sofern überhaupt notwendig erfolgt vorzugsweise mittels des oben beschriebenen Rotationstrocknungsverfahrens.

## Patentansprüche

1. Verfahren zur Beschichtung oder Befüllung der Falten eines Katheterfaltenballons, indem eine Abgabevorrichtung in Form einer Spritze, Nadel oder Düse, welche einen kontinuierlichen Fluß einer wirkstoffenthaltenden Zusammensetzung abgibt, relativ zum Katheterfaltenballon entlang der Falte bewegt wird, wobei sich der Katheterballon und die Abgabevorrichtung beide relativ zueinander bewegen.

2. Verfahren gemäß Anspruch 1, wobei Katheterfaltenballons im komprimierten oder deflatierten Zustand oder maximal 10% inflatierten Zustand beschichtet oder befüllt wird.

3. Verfahren gemäß Anspruch 1 oder 2, wobei zwei oder mehr oder alle Falten des Katheterfaltenballons gleichzeitig befüllt oder beschichtet werden.

4. Verfahren gemäß einem der vorhergehenden Ansprüche, wobei gezielt nur die Falten des Katheterfaltenballons beschichtet oder befüllt werden und die Oberfläche des Katheterfaltenballons außerhalb der Falten nicht beschichtet wird.

5. Verfahren gemäß einem der vorhergehenden Ansprüche, wobei nach der Befüllung oder Beschichtung einer oder mehrerer Falten der Katheterfaltenballon in Richtung der Faltenöffnungen rotiert wird.

6. Verfahren gemäß einem der vorhergehenden Ansprüche, wobei die Zusammensetzung mindestens einen Alkohol als Lösungsmittel enthält, ausgewählt aus der Gruppe umfassend:
Methanol, Ethanol, Iospropanol, n-Butanol, iso-Butanol, t-Butanol, Ethylenglykol, Propylenglykol, 1,3-Propandiol, Butylenglykol, 1,3-Butandiol, 1,4-Butandiol, Glycerin, 1,2,3-Butantriol, 1,2,4-Butantriol und 1,2,3,4-Butantetraol.

7. Verfahren gemäß einem der vorhergehenden Ansprüche, wobei die Zusammensetzung mindestens ein Kontrastmittel enthält, ausgewählt aus der Gruppe umfassend:
Röntgenkontrastmittel, iodhaltige Kontrastmittel, Kontrastmittel mit einem 1,3,5-Triiodbenzolkern, nephrotrope niederosmolare Röntgenkontrastmittel, Amidotrizoesäure, Iothalaminsäure, Iotrolan, Iopamidol, Iodoxaminsäure, Diatrizoesäure, Iomeprol, Iopromid, Desmethoxyacetyl-Iopromid (DAMI), 5-Amino-2,4,6-triiodphthalsäure-(2,3-dihydroxypropyl)-amid (ATH).

8. Verfahren gemäß einem der vorhergehenden Ansprüche, wobei die Zusammensetzung mindestens ein Öl und/oder mindestens eine Fettsäure enthält, ausgewählt aus der Gruppe umfassend:
Leinöl, Flachsöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran, Taririnsäure, Ximeninsäure, Stearolinsäure, 6,9-Octadeceninsäure, Pyrulinsäure, Crepenynsäure, Heisterinsäure, ETYA, Linolsäure, γ-Linolsäure, Dihomo-γ-linolensäure, Arachidonsäure, α-Linolensäure, Stearidonsäure, EPA, DPA, DHA, Meadsäure, Stellaheptaensäure, Taxolsäure, Pinolensäure, Sciadonsäure, Myristoleinsäure, Palmitoleinsäure, Petroselinsäure, Ölsäure, Vaccensäure, Gadoleinsäure, Gondoinsäure, Erucinsäure, Nervonsäure, Elaidinsäure, t-Vaccensäure, Laurinsäure, Myristinsäure, Palmitinsäure, Margarinsäure, Stearinsäure, Arachinsäure, Behensäure, Lignocerinsäure, 6,8-Dithianoctansäure, γ-Linolensäure, α-Liponsäure.

9. Verfahren gemäß einem der vorhergehenden Ansprüche, wobei die Zusammensetzung mindestens einen antiproliferativen, antiinflammatorischen, antiphlogistischen, zytostatischen, zytotoxischen, antiangiogenen, antirestenotischen oder antithrombotischen Wirkstoff umfasst, ausgewählt aus der Gruppe umfassend:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat,

Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2ω, Mitoxanthrone, Mycophenolatmofetil, c-myc-Antisense, b-myc-Antisense, β-Lapachon,Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, die Muskelzellproliferation hemmende monoklonale Antikörper, bFGF-Antagonisten, Probucol, Prostaglandine, 1-Hydroxy-11-Methoxycanthin-6-on, Scopolectin, NO-Donatoren, Pentaerythrityltetranitrat, Syndnoeimine, S-Nitrosoderivate, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel, Derivate und Analog des Paclitaxel, 6-α-Hydroxy-Paclitaxel, 2'-Succinylpaclitaxel, 2'-Succinylpaclitaxeltriethanol-amin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere, Kohlensuboxids (MCS), macrocyclische Oligomere von Kohensuboxid, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, in Virenüberträger inkorporierte Nukleinsäuren, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin, Tobramycin, Gentamycin, Penicilline, Dicloxacillin, Oxacillin, Sulfonamide, Metronidazol, Enoxoparin, desulfatiertes und N-reacetyliertes Heparin, Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon a, β und γ, Histaminantagonisten, Serotoninblocker, Apoptoseinhibitoren, Apoptoseregulatoren, p65, NF-kB, Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien-3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus, Rapamycin, Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

**10.** Verfahren gemäß Anspruch 9, wobei der mindestens eine antiproliferative, antiinflammatorische, antiphlogistische, zytostatische, zytotoxische, antiangiogene, antirestenotische oder antithrombotische Wirkstoff aus der Gruppe ausgewählt wird umfassend:
Paclitaxel, Derivate und Analog des Paclitaxel, 6-α-Hydroxy-Paclitaxel, 2'-Succinylpaclitaxel, 2'-Succinylpaclitaxeltriethanol-amin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere.

## Claims

1. Method for coating or filling the folds of a folded catheter balloon, in that a delivery device in the form of a syringe, needle or nozzle, to deliver a continuous flow of a composition including active ingredient, is moved relative to the folded catheter balloon along the fold, wherein the catheter balloon and the delivery device both move relative to each other.

2. Method according to claim 1, wherein folded catheter balloons are coated or filled in the compressed or deflated state or at maximum 10 % inflated state.

3. Method according to claim 1 or 2, wherein two or more or all of the folds of the folded catheter balloon are filled or coated simultaneously.

4. Method according to any of the preceding claims, wherein selectively only the folds of the folded catheter balloon are coated or filled and the surface of the folded catheter balloon outside the folds is not coated.

5. Method according to any of the preceding claims, wherein after the filling or coating of one or more folds the folded catheter balloon is rotated in the direction of the fold openings.

6. Method according to any of the preceding claims, wherein the composition contains at least an alcohol as solvent selected from the group comprising:
methanol, ethanol, isopropanol, n-butanol, iso-butanol, t-butanol, ethylene glycol, propylene glycol, 1,3-propanediol, butylene glycol, 1,3-butanediol, 1,4-butanediol, glycerin, 1,2,3-butanetriol, 1,2,4-butanetriol and 1,2,3,4-butanetetraol.

7. Method according to any of the preceding claims, wherein the composition contains at least a contrast agent selected from the group comprising:
x-ray contrast agents, iodine-containing contrast agents, contrast agents having a 1,3,5-triiodobenzene nucleus, nephrotropic x-ray contrast agents having low osmolarity, amidotrizoic acid, iothalaminic acid, iotrolan, iopamidol, iodoxaminic acid, diatrizoic acid, iomeprol, iopromid, desmethoxyacetyl-iopromide (DAMI), 5-amino-2,4,6-triiodophthalic acid-(2,3-dihydroxypropyl)-amide (ATH).

8. Method according to any of the preceding claims, wherein the composition contains at least an oil and/or at least a fatty acid selected from the group comprising:
linseed oil, flax oil, hempseed oil, corn oil, walnut oil, rape oil, soy bean oil, sunflower oil, poppy-seed oil, safflower oil, wheat germ oil, thistle oil, grape-seed oil, evening primrose oil, borage oil, black cumin oil, algae oil, fish oil, cod-liver oil, taririnic acid, ximeninic acid, stearolinic acid, 6,9-octadeceninic acid, pyrulinic acid, crepenynic acid, heisterinic acid, ETYA, linoleic acid, $\gamma$-linoleic acid, dihomo-$\gamma$-linoleic acid, arachidonic acid, $\alpha$-linolenic acid, stearidonic acid, EPA, DPA, DHA, meadic acid, stellaheptaenic acid, taxolic acid, pinolenic acid, sciadonic acid, myristoleic acid, palmitoleic acid, petroselinic acid, oleic acid, vaccenic acid, gadoleinic acid, gondoinic acid, erucinic acid, nervonic acid, elaidinic acid, t-vaccenic acid, laurinic acid, myristinic acid, palmitinic acid, margarinic acid, stearinic acid, arachinic acid, behenic acid, lignocerinic acid, 6,8-dithianoctanoic acid, $\gamma$-linolenic acid, $\alpha$-liponic acid.

9. Method according to any of the preceding claims, wherein the composition contains at least one antiproliferative, antiinflammatory, antiphlogistic, cytostatic, cytotoxic, antiangiogenic, anti-restenotic or antithrombotic active agent, selected from the group comprising:
abciximab, acemetacin, acetylvismione B, aclarubicin, ademetionine, adriamycin, aescin, afromoson, akagerine, aldesleukin, amidorone, aminoglutethemide, amsacrine, anakinra, anastrozole, anemonin, anopterine, antimycotics, antithrombotics, apocymarin, argatroban, aristolactam-All, aristolochic acid, ascomycin, asparaginase, aspirin, atorvastatin, auranofin, azathioprine, azithromycin, baccatine, bafilomycin, basiliximab, bendamustine, benzocaine, berberine, betulin, betulinic acid, bilobol, biolimus, bisparthenolidine, bleomycin, bombrestatin, boswellic acids and derivatives thereof, bruceanoles A, B and C, bryophyllin A, busulfan, antithrombin, bivalirudin, cadherins, camptothecin, capecitabine, o-carbamoyl-phenoxy-acetic acid, carboplatin, carmustine, celecoxib, cepharanthin, cerivastatin, CETP inhibitors, chlorambucil, chloroquine phosphate, cictoxin, ciprofloxacin, cisplatin, cladribine, clarithromycin, colchicine, concanamycin, coumadin, C-type natriuretic peptide (CNP), cudraisoflavone A, curcumin, cyclophosphamide, cyclosporine A, cytarabine, dacarbazine, daclizumab, dactinomycin, dapson, daunorubicin, diclofenac, 1,11-dimethoxycanthin-6-one, docetaxel, doxorubicin, dunaimycin, epirubicin, epothilones A and B, erythromycin, estramustine, etoposide, everolimus, filgrastim, fluoroblastin, fluvastatin, fludarabine, fludarabine-5'-dihydrogenphosphate, fluorouracil, folimycin, fosfestrol, gemcitabine, ghalakinoside, ginkgol, ginkgolic acid, glycoside 1A, 4-hydroxyoxycyclophosphamide, idarubicin, ifosfamide, josamycin, lapachol, lomustine, lovastatin, melphalan, midecamycin, mitoxantrone, nimustine, pitavastatin, pravastatin, procarbazin, mitomycin, methotrexate, mercaptopurine, thioguanine, oxaliplatin, irinotecan, topotecan, hydroxycarbamide, miltefosine, pentostatin, pegasparase, exemestane, letrozole, formestane, SMC proliferation inhibitor-2ω, mitoxantrone, mycophenolate mofetil, c-myc antisense, b-myc antisense, β-lapachone, podophyllotoxin, podophyllic acid 2-ethylhydrazide, molgramostim (rhuGM-CSF), peginterferon α-2b, lanograstim (r-HuG-CSF), macrogol, selectin (cytokine antagonist), cytokinin inhibitors, COX-2 inhibitor, NFkB, angiopeptin, monoclonal antibodies which inhibit muscle cell proliferation, bFGF antagonists, probucol, prostaglandins, 1-hydroxy-11-methoxycanthin-6-one, scopolectin, NO donors, pentaerythrityl tetranitrate, sydnone imines, S-nitroso derivatives, tamoxifen, staurosporine, β-estradiol, α-estradiol, estriol, estrone, ethinylestradiol, medroxyprogesterone, estradiol cypionates, estradiol benzoates, tranilast, kamebakaurin and other terpenoids which are applied in the therapy of cancer, verapamil, tyrosine kinase inhibitors (tyrphostins), paclitaxel, derivatives and analoga of paclitaxel, 6-α-hydroxy-paclitaxel, 2'-succinylpaclitaxel, 2'-succinyl-paclitaxeltriethanolamine, 2'-glutarylpaclitaxel, 2'-glutarylpaclitaxeltriethanolamine, 2'-O-ester of paclitaxel with N-(dimethylaminoethyl)glutamide, 2'-O-ester of paclitaxel with N-(dimethylaminoethyl)glutamide hydrochloride, taxotere, carbon suboxide (MCS), macrocyclic oligomers of carbon suboxide, mofebutazone, lonazolac, lidocaine, ketoprofen, mefenamic acid, piroxicam, meloxicam, penicillamine, hydroxychloroquine, sodium aurothiomalate, oxaceprol, β-sitosterin, myrtecaine, polidocanol, nonivamide, levomenthol, ellipticine, D-24851 (Calbiochem), colcemid, cytochalasin A-E, indanocine, nocadazole, S 100 protein, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1, active agents from the group of antibiotics, cefadroxil, cefazolin, cefaclor, cefotixin, tobramycin, gentamycin, penicillins, dicloxacillin, oxacillin, sulfonamides, metronidazole, enoxoparin, desulphated and N-reacetylated heparin, tissue plasminogen activator, GpIIb/IIIa platelet membrane receptor, factor Xa inhibitor antibody, heparin, hirudin, r-hirudin, PPACK, protamine, prourokinase, streptokinase, warfarin, urokinase, vasodilators, dipyramidol, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon a, β and γ, histamine antagonists, serotonin blockers, apoptosis inhibitors, apoptosis regulators, p65, NF-kB, Bcl-xL antisense oligonucleotides, halofuginone, nifedipine, tocopherol, tranilast, molsidomine, tea polyphenols, epicatechin gallate, epigallocatechin gallate, leflunomide, etanercept, sulfasalazine, etoposide, dicloxacillin, tetracycline, triamcinolone, mutamycin, procainimide, retinoic acid, quinidine, disopyramide, flecainide, propafenone, sotolol, natural and synthetically obtained steroids, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir, zidovudin, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceantinoside C, yadanziosides N and P, isodeoxyelephantopin, tomenphantopin A and B, coronarin A, B, C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterin, protoanemonin, cheliburin chloride, sinococuline A and B, dihydronitidine, nitidine chloride, 12-beta-hydroxypregnadien-3,20-dion, helenalin, indicine, indicine-N-oxide, lasiocarpine, inotodiol, podophyllotoxin, justicidin A and B, larreatin, malloterin, mallotochromanol, isobutyrylmallotochromanol, maquiroside A, marchantin A, maytansin, lycoridicin, margetine, pancratistatin, liriodenine, bisparthenolidine, oxoushinsunine, periplocoside A, ursolic acid, deoxypsorospermin, psycorubin, ricin A, sanguinarine, manwu wheat acid, methylsorbifolin, sphateliachromen, stizophyllin, mansonine, strebloside, dihydrousambaraensine, hydroxyusambarine, strychnopentamine, strychnophylline, usambarine, usambarensine, liriodenine, oxoushinsunine, daphnoretin, lariciresinol, methoxylariciresinol, syringaresinol, sirolimus, rapamycin, somatostatin, tacrolimus, roxithromycin, troleandomycin, simvastatin, rosuvastatin, vinblastine, vincristine, vindesine, teniposide, vinorelbine, tropfosfamide, treosulfan, temozolomide, thiotepa, tretinoin, spiramycin, umbelliferone, desacetylvismione A, vismione A and B, zeorin.

**10.** Method according to claim 9, wherein the at least one antiproliferative, antiinflammatory, antiphlogistic, cytostatic, cytotoxic, antiangiogenic, anti-restenotic or antithrombotic active agent is selected from the group comprising: paclitaxel, derivatives and analoga of paclitaxel, 6-α-hydroxy-paclitaxel, 2'-succinylpaclitaxel, 2'-succinylpaclitaxeltriethanolamine, 2'-glutarylpaclitaxel, 2'-glutarylpaclitaxeltriethanolamine, 2'-O-ester of paclitaxel with N-(dimethylaminoethyl)glutamide, 2'-O-ester of paclitaxel with N-(dimethylaminoethyl)glutamide hydrochloride, taxotere.

## Revendications

1. Procédé de revêtement ou de remplissage des plis d'un ballonnet repliable de cathéter, selon lequel un dispositif de distribution sous la forme d'une seringue, d'une aiguille ou d'une buse, qui distribue un flux continu d'une composition contenant un agent actif, est déplacé par rapport au ballonnet repliable de cathéter le long des plis, le ballonnet de cathéter et le dispositif de distribution se déplaçant tous les deux l'un par rapport à l'autre.

2. Procédé selon la revendication 1, dans lequel le ballonnet repliable de cathéter est revêtu ou rempli à l'état comprimé ou dégonflé ou au plus gonflé à 10 %.

3. Procédé selon la revendication 1 ou 2, dans lequel deux ou plus ou tous les plis du ballonnet repliable de cathéter sont remplis ou revêtus simultanément.

4. Procédé selon l'une quelconque des revendications précédentes, dans lequel uniquement les plis du ballonnet repliable de cathéter sont revêtus ou remplis de manière ciblée, et la surface du ballonnet repliable de cathéter à l'extérieur des plis n'est pas revêtue.

5. Procédé selon l'une quelconque des revendications précédentes, dans lequel le ballonnet repliable de cathéter est tourné dans la direction des ouvertures des plis après le remplissage ou le revêtement d'un ou de plusieurs plis.

6. Procédé selon l'une quelconque des revendications précédentes, dans lequel la composition contient au moins un alcool en tant que solvant, choisi dans le groupe comprenant :
le méthanol, l'éthanol, l'isopropanol, le n-butanol, l'iso-butanol, le t-butanol, l'éthylène glycol, le propylène glycol, le 1,3-propane-diol, le butylène glycol, le 1,3-butane-diol, le 1,4-butane-diol, la glycérine, le 1,2,3-butane-triol, le 1,2,4-butane-triol et le 1,2,3,4-butane-tétraol.

7. Procédé selon l'une quelconque des revendications précédentes, dans lequel la composition contient au moins un agent de contraste, choisi dans le groupe comprenant :
les agents de contraste radiographiques, les agents de contraste contenant de l'iode, les agents de contraste contenant un noyau 1,3,5-triiodobenzène, les agents de contraste radiographiques néphrotropes à faible osmolarité, l'acide amidotrizoïque, l'acide iotalamique, l'iotrolane, l'iopamidol, l'acide iodoxamique, l'acide diatrizoïque, l'ioméprol, l'iopromide, le desméthoxyacétyl-iopromide (DAMI), le (2,3-dihydroxypropyl)-amide de l'acide 5-amino-2,4,6-triiodophtalique (ATH).

8. Procédé selon l'une quelconque des revendications précédentes, dans lequel la composition contient au moins une huile et/ou au moins un acide gras, choisis dans le groupe comprenant :
l'huile de lin, l'huile de graines de lin, l'huile de chanvre, l'huile de germes de maïs, l'huile de noix, l'huile de colza, l'huile de soja, l'huile de tournesol, l'huile de pavot, l'huile de carthame (huile de carthame des teinturiers), l'huile de germes de blé, l'huile de chardon, l'huile de pépins de raisin, l'huile d'onagre, l'huile de bourrache, l'huile de nigelle, l'huile d'algue, l'huile de poisson, l'huile de foie de morue, l'acide taririque, l'acide ximénique, l'acide stéarolique, l'acide 6,9-octadécénoïque, l'acide pyrulique, l'acide crépénynique, l'acide heistérique, l'ETYA, l'acide linoléique, l'acide $\gamma$-linoléique, l'acide dihomo-$\gamma$-linolénique, l'acide arachidonique, l'acide $\alpha$-linolénique, l'acide stéaridonique, l'EPA, le DPA, le DHA, l'acide de Mead, l'acide stellaheptaénoïque, l'acide taxolique, l'acide pinolénique, l'acide sciadonique, l'acide myristoléique, l'acide palmitoléique, l'acide pétrosélinique, l'acide oléique, l'acide vaccénique, l'acide gadoléique, l'acide gondoïnique, l'acide érucique, l'acide nervonique, l'acide élaïdique, l'acide t-vaccénique, l'acide laurique, l'acide myristique, l'acide palmitique, l'acide margarique, l'acide stéarique, l'acide arachidique, l'acide béhénique, l'acide lignocérique, l'acide 6,8-dithianooctanoïque, l'acide $\gamma$-linolénique, l'acide $\alpha$-lipoïque.

9. Procédé selon l'une quelconque des revendications précédentes, dans lequel la composition comprend au moins un agent actif antiprolifératif, anti-inflammatoire, antiphlogistique, cytostatique, cytotoxique, antiangiogénique, antiresténotique ou antithrombotique, choisi dans le groupe comprenant : l'abciximab, l'acémétacine, l'acétylvismion B, l'aclarubicine, l'adémétionine, l'adriamycine, l'aescine, l'afromosone, l'akagérine, l'aldesleukine, l'amidorone, l'aminoglutéthémide, l'amsacrine, l'anakinra, l'anastrozol, l'anémonine, l'anoptérine, les antimycotiques, les antithrombotiques, l'apocymarine, l'argatroban, l'aristolactame-AII, l'acide aristolochique, l'ascomycine, l'asparaginase, l'aspirine, l'atorvastatine, l'auranofine, l'azathioprine, l'azithromycine, la baccatine, la bafilomycine, le basiliximab, la bendamustine, la benzocaïne, la berbérine, la bétuline, l'acide bétulinique, le bilobol, le biolimus, la bisparthénolidine, la bléomycine, la bombrestatine, les acides boswelliques et leurs dérivés, les brucéanols A, B et C, la bryophylline A, le busulfan, l'antithrombine, la bivalirudine, la cadhérine, la camptothécine, la capécitabine, l'acide o-carbamoylphénoxyacétique, le carboplatine, la carmustine, le célécoxib, la cépharantine, la cérivastatine, les inhibiteurs du CETP, le chlorambucil, le phosphate de chloroquine, la cictoxine, la ciprofloxacine, le cisplatine, la cladribine, la clarithromycine, la colchicine, la concanamycine, la coumadine, le peptide natriurétique de type C (CNP), la cudraisoflavone A, la curcumine, le cyclophosphamide, la cyclosporine A, la cytarabine, la dacarbazine, le daclizumab, la dactinomycine, la dapsone, la daunorubicine, le diclofénac, la 1,11-diméthoxycanthin-6-one, le docétaxel, la doxorubicine, la dunaïmycine, 1'épirubicine, les épothilones A et B, l'érythromycine, l'estramustine, l'étoboside, l'évérolimus, le filgrastim, la fluroblastine, la fluvastatine, la fludarabine, le 5'-dihydrogénophosphate de fludarabine, le fluorouracile, la folimycine, le fosfestrol, la gemcitabine, le ghalakinoside, le ginkgol, l'acide ginkgolique, le glycoside la, le 4-hydroxyoxycyclophosphamide, l'idarubicine, l'ifosfamide, la josamycine, le lapachol, la lomustine, la lovastatine, le melphalan, la midécamycine, la mitoxantrone, la nimustine, la pitavastatine, la pravastatine, la procarbazine, la mitomycine, le méthotrexate, la mercaptopurine, la thioguanine, l'oxaliplatine, l'irinotécan, le topotécan, l'hydroxycarbamide, la miltéfosine, la pentostatine, la pégasparase, l'exemestan, le létrozol, le formestan, l'inhibiteur 2ω de la prolifération des CML, les mitoxanthrones, le mycophénolatmofétil, le c-myc-antisens, le b-myc-antisens, la β-lapachone, la podophyllotoxine, le 2-éthylhydrazide de l'acide podophyllique, le molgramostim (rhuGM-CSF), le peginterféron α-2b, le lanograstim (r-HuG-CSF), le macrogol, la sélectine (antagoniste des cytokines), les inhibiteurs des cytokines, l'inhibiteur de COX-2, le NFkB, l'angiopeptine, les anticorps monoclonaux inhibiteurs de la prolifération des cellules musculaires, les antagonistes du bFGF, le probucol, les prostaglandines, la 1-hydroxy-11-méthoxycanthin-6-one, la scopolectine, les donneurs de NO, le tétranitrate de pentaérythrityle, les syndnoeimines, les dérivés S-nitrosés, le tamoxifène, la staurosporine, le β-estradiol, l'α-estradiol, l'estriol, l'estrone, l'éthynylestradiol, la médroxyprogestérone, le cypionate d'estradiol, le benzoate d'estradiol, le tranilast, la kamébakaurine et d'autres terpénoïdes qui sont utilisés dans le traitement du cancer, le vérapamil, les inhibiteurs de tyrosine-kinase (tyrphostines), le paclitaxel, les dérivés et analogues du paclitaxel, le 6-α-hydroxy-paclitaxel, le 2'-succinylpaclitaxel, la 2'-succinylpaclitaxeltriéthanolamine, le 2'-glutarylpaclitaxel, la 2'-glutarylpaclitaxeltriéthanolamine, le 2'-O-ester du paclitaxel avec le N-(diméthylaminoéthyl)glutamide, le 2'-O-ester du paclitaxel avec le chlorhydrate de N-(diméthylaminoéthyl)glutamide, les taxotères, le sous-oxyde de carbone (MCS), les oligomères macrocycliques du sous-oxyde de carbone, la mofébutazone, le lonazolac, la lidocaïne, le kétoprofène, l'acide méfénamique, le piroxicam, le méloxicam, la pénicillamine, l'hydroxychloroquine, l'aurothiomalate de sodium, l'oxacéprol, la β-sitostérine, la myrtécaïne, le polidocanol, le nonivamide, le lévomenthol, l'ellipticine, le D-24851 (Calbiochem), le colcémide, la chytochalasine A-E, les indanocines, les nocadazoles, la protéine S100, la bacitracine, les antagonistes du récepteur de la vitronectine, l'azélastine, le stimulateur de guanidylcyclase, l'inhibiteur tissulaire de la métalloprotéinase-1 et - 2, les acides nucléiques libres, les acides nucléiques incorporés dans les vecteurs viraux, les fragments d'ADN et d'ARN, l'inhibiteur-1 de l'activateur du plasminogène, l'inhibiteur-2 de l'activateur du plasminogène, les oligonucléotides antisens, les inhibiteurs du VEGF, l'IGF-1, les principes actifs du groupe des antibiotiques, le céfadroxil, la céfazoline, le céfaclor, la céfotixine, la tobramycine, la gentamycine, les pénicillines, la dicloxacilline, l'oxacilline, les sulfonamides, le métronidazol, l'énoxoparine, l'héparine désulfatée et N-réacétylée, l'activateur du plasminogène tissulaire, le récepteur membranaire plaquettaire GpIIb/IIIa, les anticorps des inhibiteurs du facteur Xa, l'héparine, l'hirudine, la r-hirudine, le PPACK, la protamine, la prourokinase, la streptokinase, la warfarine, l'urokinase, les vasodilatateurs, le dipyramidol, le trapidil, le nitroprusside, les antagonistes du PDGF, la triazolopyrimidine, la séramine, les inhibiteurs de l'ECA, le captopril, le cilazapril, le lisinopril, l'énalapril, le losartan, les inhibiteurs des thioprotéases, la prostacycline, le vapiprost, l'interféron a, β et γ, les antagonistes des histamines, les sérotoninobloquants, les inhibiteurs de l'apoptose, les régulateurs de l'apoptose, le p65, le NF-kB, les oligonucléotides Bcl-xL-antisens, l'halofuginone, la nifédipine, le tocophérol, le tranilast, la molsidomine, les polyphénols du thé, le gallate d'épicatéchine, le gallate d'épigallocatéchine, le léflunomide, l'étanercept, la sulfasalazine, l'étoposide, la dicloxacylline, la tétracycline, la triamcinolone, la mutamycine, le procaïnimide, l'acide rétinoïque, la quinidine, le disopyrimide, le flécaïnide, la propafénone, le sotolol, les stéroïdes naturels et fabriqués par synthèse, l'inotodiol, le maquiroside A, le ghalakinoside, la mansonine, le strébloside, l'hydrocortisone, la bétaméthasone, la dexaméthasone, les substances non stéroïdiennes (AINS), le fénoporfène, l'ibuprofène, l'indométhacine, le naproxène, la phénylbutazone, les agents antiviraux, l'acyclovir, le ganciclovir, la zidovudine, le clotrimazol, la flucytosine, la griséofulvine, le kétoconazol, le miconazol, la nystatine, la terbinafine, les agents antiprozoaires, la chloroquine, la méfloquine, la quinine, les terpénoïdes naturels, l'hippocaesculine, l'angélate de barringtogénol-C21, la 14-déhydroagrostistachine, l'agroskérine, l'agrostistachine, la 17-hydroxyagrostistachine, les ovatodiolides, l'acide 4,7-oxycycloanisomélique, les baccharinoïdes B1, B2, B3 et B7, le tubéimoside, les brucéantinosides C, les yadanziosides N et P, l'isodésoxyéléphantopine, la tomenphantopine A et B, la coronarine A, B, C et D, l'acide ursolique, l'acide hyptatique A, l'iso-iridogermanal, le maytenfoliol, l'effusantine A, l'excisanine A et B, la longikaurine B, la sculponéatine C, la kamébaunine, la leukaménine A et B, la 13,18-déhydro-6-alpha-sénocioyloxychaparrine, la taxamairine A et B, le régénilol, le triptolide, la cymarine, l'hydroxyanoptérine, la protoanémonine, le chlorure de chéliburine, la sinococuline A et B, la dihydronitidine, le chlorure de nitidine, la 12-bêta-hydroxypregnadièn-3,20-dione, l'hélénaline, l'indicine,

le N-oxyde d'indicine, la lasiocarpine, l'inotodiol, la podophyllotoxine, la justicidine A et B, la larréatine, la mallotérine, le mallotochromanol, l'isobutyrylmallotochromanol, le maquiroside A, la marchantine A, la maytansine, la lycoridicine, la margétine, la pancratistatine, la liriodénine, la bisparthénolidine, l'oxoushinsunine, le périplocoside A, l'acide ursolique, la désoxypsorospermine, la psycorubine, la ricine A, la sanguinarine, l'acide manwuwéizique, la méthylsorbifoline, le sphathéliachromène, la stizophylline, la mansonine, le strébloside, la dihydrousambaraensine, l'hydroxyusambarine, la strychnopentamine, la strychnophylline, l'usambarine, l'usambarensine, la liriodénine, l'oxoushinsunine, la daphnorétine, le laricirésinol, le méthoxylaricirésinol, le syringarésinol, le sirolimus, la rapamycine, la somatostatine, le tacrolimus, la roxithromycine, la troléandomycine, la simvastatine, la rosuvastatine, la vinblastine, la vincristine, la vindésine, le téniposide, la vinorelbine, le tropfosfamide, le tréosulfan, le trémozolomide, le thiotépa, la trétinoïne, la spiramycine, l'umbelliférone, la désacétylvismione A, la vismione A et B, la zéorine.

**10.** Procédé selon la revendication 9, dans lequel ledit au moins un agent actif antiprolifératif, anti-inflammatoire, antiphlogistique, cytostatique, cytotoxique, antiangiogénique, antiresténotique ou antithrombotique est choisi dans le groupe comprenant : le paclitaxel, les dérivés et analogues du paclitaxel, le 6-$\alpha$-hydroxy-paclitaxel, le 2'-succinylpaclitaxel, la 2'-succinylpaclitaxeltriéthanolamine, le 2'-glutarylpaclitaxel, la 2'-glutarylpaclitaxeltriéthanolamine, le 2'-O-ester du paclitaxel avec le N-(diméthylaminoéthyl)glutamide, le 2'-O-ester du paclitaxel avec le chlorhydrate de N-(diméthylaminoéthyl)glutamide, les taxotères.

Figur 1

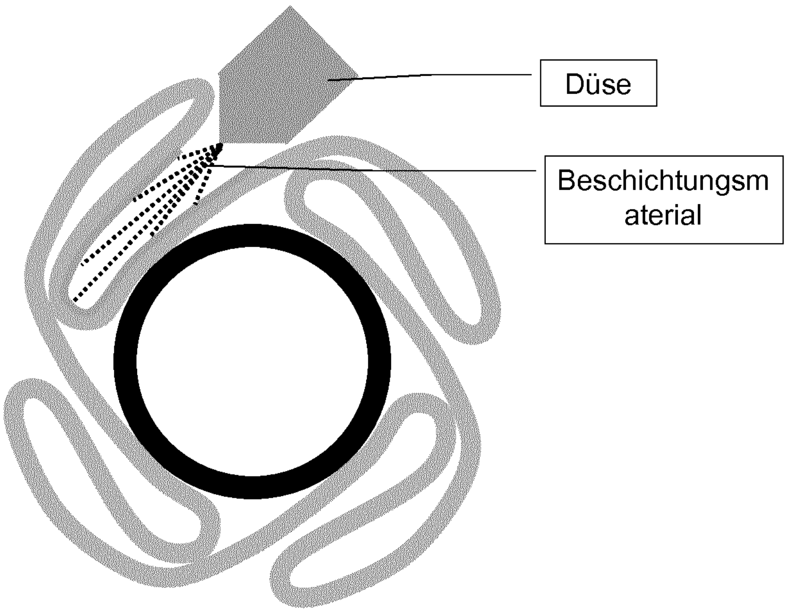


Figur 2

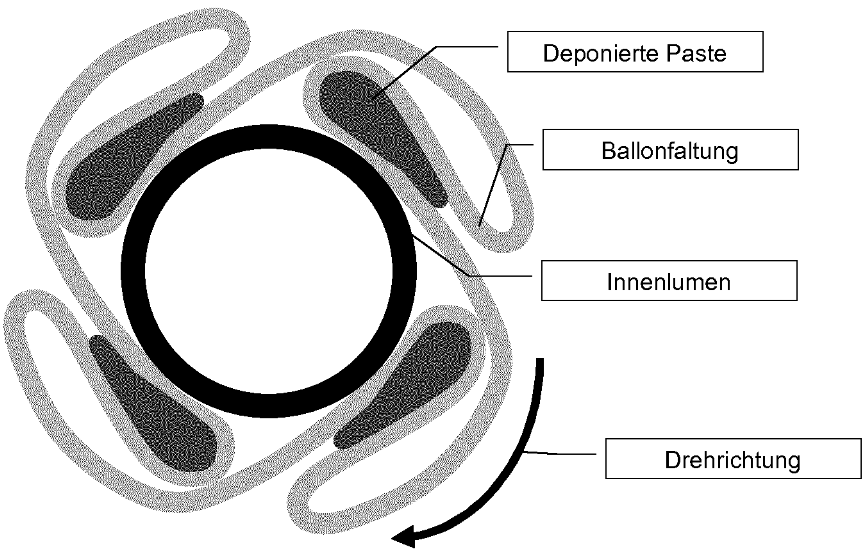

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20040071861 A
- WO 03035131 A
- WO 0245744 A
- EP 0519063 B1
- WO 2004028582 A1
- WO 2004028610 A2
- WO 2008086794 A2
- WO 9423787 A1
- WO 03059430 A1
- WO 03022265 A1